# EUROPÄISCHE PATENTSCHRIFT

(11) **EP 3 445 816 B1**
(45) Veröffentlichungstag und Bekanntmachung des Hinweises auf die Patenterteilung: **29.01.2020**
(21) Anmeldenummer: 17721551.4
(22) Anmeldetag: 19.04.2017
(51) Int. Cl.: C08L 25/12, C08L 79/02, C08L 55/02, C08G 73/02, A01N 47/44, A61K 31/785

(54) **ANTIMIKROBIELL WIRKENDE, NICHT AUSBLUTENDE THERMOPLASTISCHE FORMMASSEN**
ANTIMICROBIALLY ACTIVE, NON-LEACHING THERMOPLASTIC MOLDING COMPOUNDS
MATIÈRES À MOULER THERMOPLASTIQUES NON PÉNÉTRANTES À EFFET ANTIMICROBIEN

(30) Priorität: 20.04.2016 EP 16166122
(43) Veröffentlichungstag der Anmeldung: 27.02.2019
(73) Patentinhaber: INEOS Styrolution Group GmbH, 60325 Frankfurt (DE)
(72) Erfinder: BENECKE, Bianca, 63456 Hanau (DE); NIESSNER, Norbert, 67159 Friedelsheim (DE); GREINER, Andreas, 95444 Bayreuth (DE); AGARWAL, Seema, 95444 Bayreuth (DE); BENKER, Lothar, 95447 Bayreuth (DE)
(74) Vertreter: Jacobi, Markus Alexander
(86) Internationale Anmeldenummer: PCT/EP2017/059312
(87) Internationale Veröffentlichungsnummer: WO 2017/182536

(56) Entgegenhaltungen:
- EP-A1- 1 486 519
- WO-A1-2011/058144
- WO-A1-2011/131773
- KR-B1- 100 851 374
- US-B1- 6 225 417
- DATABASE WPI Week 201150 Thomson Scientific, London, GB; AN 2011-G53115 XP002771798, -& CN 102 040 771 A (CHINA PETROCHEMICAL CO LTD) 4. Mai 2011 (2011-05-04)

## Beschreibung

Die Erfindung betrifft thermoplastische Formmassen M, enthaltend eine antimikrobiell wirksame Polymerkomponente A, enthaltend 0,2 bis 25 Gew.-%, bezogen auf die Gesamtmasse an Polymerkomponente A, an Polyguanidin A1, und eine oder mehrere Polymere A2, welche eine oder mehrere funktionelle Gruppen tragen, die mit Polyguanidin A1 reagieren können. Ferner betrifft die vorliegende Erfindung ein Verfahren zur Herstellung einer antimikrobiell wirksamen thermoplastischen Formmasse M, umfassend den Schritt des Extrudierens einer Polyguanidin-Komponente und mindestens eines Polymers A2. Zudem bezieht sich die Erfindung auf Formteile und andere Produkte, die die thermoplastische Formmasse M enthalten, sowie auf die Verwendung der thermoplastischen Formmasse M.

Zur antimikrobiellen Ausrüstung von Kunststoffen, insbesondere thermoplastischen Copolymer-Formmassen, bieten sich grundsätzlich zwei Arten von Additiven an, anorganische und organische Komponenten. Viele dieser Komponenten sind bekannt.

Anorganische antimikrobielle Additive basieren beispielsweise auf Metallionen, z.B. von Silber, Kupfer, Nickel oder Zink. Sie zeigen im Allgemeinen zwar eine gute antimikrobielle Wirkung, besitzen aber einige Nachteile, wie etwa ein mögliches Verursachen von Verfärbungen im Kunststoff oder das Ausbluten (engl. "Leaching") der Metallionen. Aufgrund des Ausblutens verliert das Polymer die antimikrobielle Ausstattung. Der fortgesetzte Kontakt zu Silber-Ionen kann auch chronische Vergiftungserscheinungen hervorrufen. Auch Kupfer-, Nickel- und Zink-Ionen können toxisch und sein und sind unerwünscht. Zudem kann es zu Verfärbungen durch Redoxreaktionen der Metallionen kommen.

In WO 2014/155156 werden antibakterielle Polymere beschrieben, bei denen Zinksalze während der Polymerisation zugegeben werden. Dadurch sollen die Zink-Ionen besser in der Matrix gebunden sein und dennoch antibakteriell wirken. Obwohl die Anbindung besser als bei einer reinen Mischung von Polymer und Zinksalz ist, kommt es dennoch zu einem messbaren Ausbluten der Zink-Ionen. Bereits nach 2 Stunden bei 70°C liegt die Zink-Migration aus dem Copolymer ABS im wässrigen Medium und in Essigsäure schon bei 0,33 ppm.

WO 2015/040642 beschreibt ein Zink-haltiges antibakterielles Material für die Anwendung im Lebensmittel-Kontaktbereich. Ein Zink-Pyrrolidon-carboxylat wird co-extrudiert mit einem Polymer. Der Nachteil dieses Vorgehens ist, dass es sich um eine Mischung des antimikrobiellen Wirkstoffs und einer Polymermatrix handelt. Ein Ausbluten des Wirkstoffs kann nicht verhindert werden.

Organische antimikrobielle Additive zeigen häufig bereits bei relativ geringen zugesetzten Mengen eine merkliche antimikrobielle Wirkung. Auch diese weisen jedoch oft noch technische Nachteile auf. Beispielsweise besteht bei manchen organischen antimikrobiellen Additiven die Gefahr der thermischen Zersetzung bei der Verarbeitung der Kunststoffe, so dass die antimikrobielle Wirkung vermindert wird oder ganz verloren gehen kann. Ein weiteres Problem besteht in der Migration kleinerer organischer Moleküle an die Kunststoffoberfläche der Formteile und das darauf folgende Ausbluten des Wirkstoffes.

EP-B 1280766 beschreibt antimikrobielle Polymere auf der Basis von Guanidin-Salzen mit hoher Aktivität und geringer Toxizität. Es wird jedoch nicht versucht, diese Guanidin-Polymere mit anderen Kunststoffen zu einer Formmasse zu vermengen (zu compoundieren) oder die Löslichkeit der Guanidin-Polymere zu verringern. Im Gegenteil sind die darin beschriebenen antimikrobiellen Polymere durchweg in Wasser löslich, was dem Ziel des Verhinderns von Ausbluten entgegengesetzt ist.

In DE-A 102004011293 wird die Verwendung von Polyhexamethylenbiguanidin (PHMB) genannt, welches in Lösung gebracht auf ein Medizinprodukt aufgebracht wird. PHMB ist ein vergleichsweise wirksames, unspezifisch gegen viele Bakterien einsetzbares antimikrobielles Mittel, welches aber oberflächlich aufgebracht den Nachteil hat, leicht abgewaschen werden zu können. Eine Verzögerung des Abwaschens wird durch eine Deckschicht aus einem weiteren Material mit kleinen Durchtrittsöffnungen erreicht, was aber wiederum den Nachteil hat, dass ein weiterer Arbeitsschritt erforderlich ist. Zudem wird das Auswaschen nur verzögert, aber nicht verhindert. Für eine Langzeit-Ausrüstung eines Gegenstands mit dem antimikrobiellen Additiv ist diese Ausführungsform daher ungeeignet.

EP-A 1110948 beschreibt die Verwendung von Polyhexamethylenguanidinphosphat in antimikrobiell wirkenden Zusammensetzungen. Diese Verbindung ist bei den üblichen Prozesstemperaturen der Kunststoffverarbeitung stabil und zeigt weniger Neigung zur Migration als andere niedermolekulare organische Verbindungen. Der Nachteil von Polyhexamethylenguanidinphosphat ist, dass es nicht in flüssiger Form dem Prozess zugesetzt werden kann, sondern als Pulver zum Polymer hinzugegeben werden muss. Zwar lässt sich das so eingemischte Polyhexamethylenguanidinphosphat schon deutlich weniger auswaschen als niedermolekulare organische antimikrobielle Verbindungen, jedoch ist immer noch ein erhebliches Auswaschen feststellbar.

EP-A 2230258 beschreibt die Verwendung eines Gemisches aus einem Matrixpolymer und einem antimikrobiell wirkenden polymeren Guanidinderivat als antimikrobielles Mittel, wobei jedoch keine Anbindung des Guanidins an die Matrix erfolgt und auch das Ausblut-Verhalten nicht geprüft wird, da es sich um ein oral zu verabreichendes Arzneimittel zur Behandlung von Infektionen des Gastrointestinaltraktes in der Humanmedizin und in der Veterinärmedizin handelt.

In WO 2009/009814 wird ein Polymer beschrieben, welches ein mit einem polymeren Guanidinderivat modifiziertes Silikat enthält und als Dentalfüllstoff dienen soll. Trotz Immobilisierung an das Silikat besitzt das Guanidinderivat noch eine antimikrobielle, plaquehemmende Wirkung. Die verwendeten Kunststoffe beschränken sich auf härtbare Kunststoffe, bevorzugt auf Acrylat- oder Methacrylatbasis. Ein Nachteil dieser Erfindung ist jedoch, dass der Wirkstoff an Silkat gebunden ist; für transparente Anwendungen ist die beschriebene Methode somit ungeeignet. Die Erfindung ist auch auf eine geringe Auswahl an Polymeren begrenzt. Das Eluat enthält zudem messbare Mengen an Wirkstoff, die aus dem Material herausgewaschen wurden.

WO 2011/057611 stellt ein Verfahren zur Erzeugung polymerer und oligomerer Polyguanidine mit antimikrobieller Wirkung bereit. Die Wirkstoffe werden unter anderem in Zusammensetzungen in Form von Kunststoffgranulaten erzeugt. Der Wirkstoff ist dabei kovalent an ein Matrixpolymer gebunden. Im Fall von thermoplastischem aliphatischem oder thermoplastischem aliphatischem/aromatischem Polyurethan bildet sich bei der Compoundierung eine Formmasse M aus den vermischten Komponenten. Dazu sind jedoch reaktive Gruppen im Polymer nötig, beispielsweise Isocyanatgruppen. Das Verfahren ist daher nicht für alle thermoplastischen Kunststoffe geeignet, insbesondere nicht für Kunststoffe auf Acrylnitril-Styrol-Basis wie ABS oder ASA, die aber aufgrund ihrer positiven Produkteigenschaften, wie z.B. hervorragende Oberflächengüte, für viele Anwendungen präferiert werden.

US 5,142,010 und CN-A 101628952 beschreiben die Herstellung von antimikrobiell wirksamen Polyguanidinen, es fehlt jedoch eine Anbindung des Guanidinpolymers an eine Matrix, insbesondere fehlen Mengenanteile an brauchbaren Co-Monomeren zur kovalenten Anbindung von organischen antimikrobiellen Mitteln.

Die vorgenannten Dokumente des Standes der Technik stellen zwar antimikrobielle Kunststoffe bereit, jedoch fehlt eine angemessene Anbindung der Wirkkomponente an die Matrix. Diese Fixierung sollte so sein, dass es zu vernachlässigbar geringem Ausbluten kommt, insbesondere in Verbindung mit Kunststoffen, die wünschenswerte mechanische und optische Eigenschaften besitzen, wie etwa Copolymere auf Styrol-Acrylnitril-Basis, wie z.B. ABS oder ASA-Copolymere.

WO 2011/058144 beschreibt die Verwendung von Polyguanidinen in medizinischen Artikeln in Verbindung mit thermoplastischen Polymeren ausgewählt aus der Gruppe Polyurethan, Polycarbonat, Polyethersulfon, Silikon, Polyester, Polyamid, Polycaprolactam, Polylactid und Polyharnstoff, wobei diese mit dem antimikrobiellen Wirkstoff eine kovalente Verknüpfung eingehen. Dazu ist es notwendig, dass im thermoplastischen Polymer noch reaktive Gruppen, z.B. Isocyanat-Gruppen, vorhanden sind, oder dass, wie im Fall der Polyurethane, eine Umkondensations-Reaktion möglich ist. Der oligomere oder polymere antimikrobielle Wirkstoff wird bevorzugt gelöst in einer Flüssigkeit zugegeben. Dieses Vorgehen ist vergleichsweise aufwendig und Nebenreaktionen können auf Grund der Reaktivität der Seitengruppen teilweise nicht ausgeschlossen werden.

WO 2011/131773 beschreibt den Einsatz spezifischer Polyguanidinsalze in Polymeren wie Polyamiden und Poly(venyl)fluoriden. CN-A 102040771 beschreibt den Einsatz von Polyguanidin-Polysilikat-Verbindungen als antibakterielle Mittel, die Polymeren beigemischt werden können.

EP-A 784080 beschreibt die Verwendung eines Styrol-Acrylnitril-Maleinsäureanhydrid-Terpolymers zur Kompatibilitätsverbesserung zwischen Polyamid und ABS. Es wird davon ausgegangen, dass die Amino- bzw. Carboxyl-Endgruppen der Polyamide mit den funktionellen Gruppen der Terpolymere reagieren, wobei *in situ* Copolymere entstehen, welche die Verträglichkeit zwischen der Styrolcopolymer-Phase und der Polyamid-Phase herstellen. EP-A 784080 beschreibt jedoch keine antimikrobielle Ausrüstung von Copolymeren.

Es besteht demnach das Bedürfnis, antimikrobiell wirksame thermoplastische Formmassen, insbesondere solche auf Styrol-Acrylnitril-Copolymer-Basis, mit verringertem Ausblut-Verhalten bereitzustellen. Verringertes Ausblut-Verhalten bedeutet dabei, dass bei Kontakt des antimikrobiell wirksam ausgerüsteten thermoplastischen Materials mit flüssigen Medien, wie Wasser, Körperflüssigkeiten oder Reinigungsflüssigkeiten, der antimikrobielle Wirkstoff selbst nicht bzw. nur in äußerst geringen Mengen (und über einen langen Zeitraum) freigesetzt wird. Weitere wünschenswerte Eigenschaften sind, dass die antimikrobiell wirksame thermoplastische Formmasse gut thermoplastisch zu verarbeiten ist und nicht stark durch den antimikrobiellen Wirkstoff verfärbt wird.

Überraschend wurde gefunden, dass thermoplastische Formmassen M, die Polyguanidin als antimikrobiell wirksame Komponente und mindestens ein Polymer A2, enthaltend mindestens eine funktionelle Gruppe, welche mit A1 reagieren kann (beispielhaft Maleinsäureanhydrid-haltige Copolymere) enthalten, eine gute antimikrobielle Wirkung und gleichzeitig geringes Ausbluten zeigen. Während des Compoundierens scheint eine Reaktion zwischen den Polyguanidin-Oligomeren (bzw. Polymeren) und dem Polymer A2 zu erfolgen, so dass eine kovalente Anbindung der Polyguanidine an die Copolymer-Komponente der thermoplastischen Formmassen M stattfindet.

Auch bei vergleichsweise geringen Anteilen an funktionellen Gruppen (wie etwa Maleinsäureanhydrid) und einem vergleichsweise hohen Gehalt an Polyguanidin wurde ein sehr geringes Ausbluten beobachtet, obwohl ein erheblicher Anteil an nicht-reagiertem Polyguanidin erwartet werden kann. Insbesondere die Verwendung von thermoplastischen Formmassen M auf Styrol-Acrylnitril-Copolymer-Basis, wie z.B. Styrol-Acrylnitril-Maleinsäureanhydrid-Terpolymer als Polymer A2, zeigte hierbei ausgesprochen vorteilhafte Eigenschaften. Das Polymer A2 konnte auch mit anderen thermoplastischen (Co)polymeren compoundiert werden und besaß auch bei größerer Verdünnung in der Zusammensetzung noch gute mechanische, optische und antimikrobielle Eigenschaften.

Gelehrt wird eine thermoplastische Formmasse M bestehend aus:
(A) 1 bis 100 Gew.-%, insbesondere 2 bis 80 Gew.-%, (mindestens) einer antimikrobiell wirksamen Polymerkomponente A, enthaltend (oder bestehend aus)
   (A1) 0,2 bis 25 Gew.-%, bezogen auf die Gesamtmasse an Polymerkomponente A, an Polyguanidin als Komponente A1 und
   (A2) 75-99,8 Gew.-%, bezogen auf die Gesamtmasse an Polymerkomponente A, an (mindestens einem) Polymer A2, enthaltend mindestens eine funktionelle Gruppe, welche mit A1 reagieren kann;
(B) 0 bis 99 Gew.-%, insbesondere 20 bis 98 Gew.-%, einer weiteren thermoplastischen Polymerkomponente B; und
(C) 0 bis 40 Gew.-%, insbesondere 0,2 bis 15 Gew.-%, oftmals 0,2 bis 5 Gew.-%, eines oder mehrerer Additive oder Hilfsmittel als Komponente C,
wobei die Summe der Gew.-% der Komponenten A, B und C zusammen 100 Gew.-% ergibt.

Erfindungsgemäß ist das (mindestens eine) Polymer A2 hierbei ein Copolymer aus:
A21: Maleinsäureanhydrid (MA), Maleinimid und/oder (Meth)acrylsäure, und
A22: einem oder mehreren der Monomere Styrol, Acrylnitril, (Meth)acrylat, und
A23: ggf. einem oder mehreren weiteren mit A21 und A22 co-polymerisierbaren Monomeren.

Die vorliegende Erfindung ist gemäß den Ansprüchen definiert.

Ein Fachmann versteht gemäß allgemeinen Fachverständnisses alle hierin angegebenen prozentualen Werte als auf die angegebene Nachkommastelle gerundete Werte, daher beispielsweise "80 Gew.-%" als "79,5 bis 80,4 Gew.-%". Da die Summe der Komponenten A, B und C zusammen 100 Gew.-% ergeben soll, wird der Fachmann entsprechende Rundungen vornehmen. Beispiele hierfür, die nicht einschränkend zu verstehen sind, sind hierin angegeben. Die Komponente A ist von B verschieden.

Gemäß einer bevorzugten Ausführungsform umfasst die thermoplastische Formmasse M demnach (oder besteht aus):
(A) 2 bis 80 Gew.-% (beispielsweise 2 bis 79,8 Gew.-%), (mindestens) einer antimikrobiell wirksamen Polymerkomponente A, enthaltend (oder bestehend aus) (A
   1) 0,2 bis 25 Gew.-%, bezogen auf die Gesamtmasse an Polymerkomponente A, an Polyguanidin als Komponente A1 und
   (A2) 75-99,8 Gew.-%, bezogen auf die Gesamtmasse an Polymerkomponente A, an (mindestens einem) Polymer A2, wobei das Polymer A2 erfindungsgemäß ein Copolymer ist aus:
      A21: Maleinsäureanhydrid (MA), Maleinimid und/oder (Meth)acrylsäure, und
      A22: einem oder mehreren der Monomere Styrol, Acrylnitril, (Meth)acrylat, und
      A23: ggf. einem oder mehreren weiteren mit A21 und A22 co-polymerisierbaren Monomeren,
      enthaltend mindestens eine funktionelle Gruppe, welche mit A1 reagieren kann;
(B) 20 bis 98 Gew.-% (beispielsweise 20 bis 97,8 Gew.-%) einer weiteren thermoplastischen Polymerkomponente B; und
(C) 0,2 bis 15 Gew.-%, oftmals 0,2 bis 5 Gew.-%, eines oder mehrerer Additive oder Hilfsmittel als Komponente C,
wobei die Summe der Gew.-% der Komponenten A, B und C zusammen 100 Gew.-% ergibt.

Daher kann die thermoplastische Formmasse M (weitgehend) aus der antimikrobiell wirksamen Polymerkomponente A oder aus den Komponenten A und B bestehen.

Das Polymer A2 ist bevorzugt eine thermoplastische Polymer-Komponente. Das Polymer A2 kann eine beliebige Größe, daher ein beliebiges Molekulargewicht (Mw) aufweisen. Gemäß einer bevorzugten Ausführungsform ist das Polymer A2 ein Polymer mit mindestens 1000 Dalton mittlerem Molekulargewicht.

Es wird gelehrt, dass das Polymer A2 eine beliebige funktionelle Gruppe, welche mit A1 reagieren kann, enthalten kann. Daher wird gelehrt, dass das Polymer A2 eine beliebige Funktionalität enthalten kann, die eine kovalente oder nicht-kovalente Bindung an Komponente A1 ermöglicht. Es wird gelehrt, dass das Polymer A2 eine oder mehrere der folgenden Funktionalitäten: Anhydrid, Säure, Base, Aminogruppe enthalten kann.

Erfindungsgemäß ist das (mindestens eine) Polymer A2 ein Copolymer aus:
A21: Maleinsäureanhydrid (MA), Maleinimid und/oder (Meth)acrylsäure, und
A22: einem oder mehreren der Monomere Styrol, Acrylnitril, (Meth)acrylat, und
A23: ggf. einem oder mehreren weiteren mit A21 und A22 co-polymerisierbaren Monomeren.

Gemäß einer bevorzugten Ausführungsform enthält das Polymer A2 ein oder mehrere Anhydrid(e). Besonders bevorzugt enthält das Polymer A2 Maleinsäureanhydrid (MA).

Daher enthält das Polymer A2 besonders bevorzugt ein Copolymer aus:
A21: Maleinsäureanhydrid (MA), und
A22: einem oder mehreren der Monomere Styrol, Acrylnitril, (Meth)acrylat, und
A23: ggf. einem oder mehreren weiteren mit A21 und A22 copolymerisierbaren Monomeren.

Häufig ist das Polymer A2 ein Styrol-Copolymer, das Maleinsäureanhydrid (MA) enthält. Noch stärker bevorzugt ist das Polymer A2 ein Copolymer bestehend aus:
A21: Maleinsäureanhydrid (MA), und
A22: Styrol und
A23: ggf. einem oder mehreren weiteren mit A21 und A22 copolymerisierbaren Monomeren, wie etwa Acrylnitril und/oder Meth)acrylat.

Gemäß einer bevorzugten Ausführungsform ist das Polymer A2 ein Styrol-Acrylnitril-Maleinsäureanhydrid-Terpolymer-Konjugat (SAN MA). Dann kann die Polymerkomponente A auch als Polyguanidin-Styrol-Acrylnitril-Maleinsäureanhydrid-Terpolymer-Konjugat (PG/SAN MA) bezeichnet werden.

Wird in der thermoplastischen Formmasse M neben Polymerkomponente A (besonders bevorzugt PG/SAN MA) noch mindestens eine weitere thermoplastische Polymerkomponente B eingesetzt, kann das Konjugat aus Polyguanidin und Polymer A2 auch als Masterbatch eingesetzt werden.

Die thermoplastische Formmasse M kann optional als Granulat (Kunststoffgranulat) vorliegen und so zur weiteren Verarbeitung zur Verfügung stehen. Die erfindungsgemäße thermoplastische Formmasse M (insbesondere wenn diese in Form eines Granulats vorliegt) kann auch in Form eines Masterbatches bereitgestellt werden.
Dieses kann dann vor der entsprechenden Verwendung nochmals (etwa mit einer oder mehreren anderen (vorzugsweise) thermoplastischen Formmasse(n), z.B. auch in Granulatform) verdünnt werden. Alternativ kann die thermoplastische Formmasse M auch in Form eines Formteils F und/oder eines Produkts vorliegen.

Der Fachmann versteht, dass mit "Styrol" (S), "Acrylnitril" (AN), "Maleinsäureanhydrid" (MA), "Maleinimid" und/oder "(Meth)acrylsäure", "Butadien" (B) usw. die in die jeweilige (Co)polymer-Struktur eingebetteten Einheiten gemeint sind.

Die antimikrobiell wirksamen Polymerkomponente A kann 0,2 bis 25 Gew.-%, bezogen auf die Gesamtmasse an Polymerkomponente A, an Polyguanidin als Komponente A1 enthalten. In der Polymerkomponente A beträgt der Anteil an Polyguanidin bevorzugt weniger als 15 Gew.-%, ganz besonders bevorzugt weniger als 10 Gew.-%.

Gemäß einer bevorzugten Ausführungsform enthält die antimikrobiell wirksame Polymerkomponente A von 0,2 bis 15 Gew.-%, bezogen auf die die Gesamtmasse der Polymerkomponente A, an Polyguanidin als Komponente A1. Gemäß einer stärker bevorzugten Ausführungsform enthält die antimikrobiell wirksame Polymerkomponente A 0,2 bis 10 Gew.-%, bezogen auf die die Gesamtmasse der Polymerkomponente A, an Polyguanidin als Komponente A1.

Bevorzugt enthält die thermoplastische Formmasse M 0,2 bis 15 Gew.-%, bevorzugt 0,2 bis 10 Gew.-%, insbesondere 0,5 bis 5 Gew.-%, bezogen auf die die Gesamtmasse an thermoplastischer Formmasse M, an Polyguanidin (als Komponente A1). Beispielsweise kann die thermoplastische Formmasse M 0,5 bis 1,5 Gew.-%, 1 bis 2 Gew.-%, 1,5 bis 3 Gew.-%, 2 bis 4 Gew.-%, 3 bis 5 Gew.-% oder 4 bis 10 Gew.-%, bezogen auf die die Gesamtmasse an thermoplastischer Formmasse M, an Polyguanidin (als Komponente A1) enthalten.

Im Sinne der vorliegenden Erfindung kann ein beliebiges Polyguanidin als Komponente A1 eingesetzt werden. Der Begriff "Polyguanidin" ist im weitesten Sinne als Reaktionsprodukt aus einem oder mehreren Guanidinsalzen (z.B. Guanidinhydrochlorid oder Guanidinsulfat) mit einem oder mehreren aliphatischen Diamin(en) (beispielsweise 1,6-Hexamethylendiamin (HMDA)) zu verstehen. Derartige Produkte sind dem Fachmann bekannt.

Bevorzugt enthält ein aliphatisches Diamin im Sinne der vorliegenden Erfindung keinen Cyloalkyl- bzw. Cycloalkylen-Rest, sondern basiert auf einem oder mehreren linearen Alkyldiaminen, etwa C₁-C₁₀-Alkyldiaminen, bevorzugt C₂-C₈-Alkyldiaminen, stärker bevorzugt C₄-C₈-Alkyldiaminen. Bevorzugt sind hierbei die terminalen Kohlenstoffatome mit jeweils einer Aminogruppe versehen.

Daher ist das aliphatische Diamin bevorzugt ein solches der Formel NH₂(CH₂)ₙNH₂, wobei n = 1, 2, 3, 4, 5, 6, 7, 8, 9 oder 10, insbesondere 4, 5, 6, 7 oder 8, ist. Gemäß einer stark bevorzugten Ausführungsform wird das als Komponente A1 eingesetzte Polyguanidin durch Reaktion von Guanidinhydrochlorid (GHC) mit 1,6-Hexamethylendiamin (HMDA) erhalten.

Alternativ zu Chloriden und Sulfaten können auch andere Salze, die ein Guanidin-Ion enthalten (beispielsweise Carbonate, Acetate, Benzoate, Hydroxide, Hydrogensulfate etc.) verwendet werden.
Bei Reaktionen dieses Typs bilden sich vorzugsweise Oligomere oder polymere Polykondensate. Bevorzugt weisen die erfindungsgemäß einzusetzenden Polyguanidine ein mittleres Molekulargewicht Mₙ von 500 bis 10.000, stärker bevorzugt von 2000 bis 7.000 auf. Gemäß einer bevorzugten Ausführungsform weist das als Komponente A1 eingesetzte Polyguanidin daher ein Molekulargewicht Mₙ von 2.000 bis 10.000, insbesondere 2.000 bis 7.000 auf.

Die erfindungsgemäß verwendeten Polyguanidine zeigen eine antimikrobielle Wirkung. Diese antimikrobielle Wirkung ist im weitesten Sinne als das Abtöten und/oder die Proliferationshemmung von Mikroorganismen zu verstehen. Dem Fachmann sind zahlreiche Möglichkeiten bekannt, eine solche Wirkung nachzuweisen. Beispielsweise kann eine solche antimikrobielle Wirkung mittels einer minimalen Hemmkonzentration (engl. "Minimum Inhibitory Concentration", MIC) beschrieben werden. Die MIC gibt die niedrigste Konzentration des antimikrobiellen Mittels an, die das Wachstum von Bakterien in einer bestimmten Lösung hemmt. Bevorzugt sind solche Polyguanidine, die eine MIC von weniger als 50 µg/mL, stärker bevorzugt von weniger als 25 µg/mL, insbesondere von weniger als 10 µg/mL aufweisen. Je geringer die minimale Hemmkonzentration ist, desto weniger antimikrobielles Mittel wird für eine entsprechende Wirkung benötigt, und umso effektiver kann das antimikrobielle Agens eingesetzt werden.

Eine zusätzliche oder alternative Testmethode zur Bestimmung der antimikrobiellen Wirkung ist die Bestimmung der minimalen Bakterienkonzentration (engl. "Minimum Bacterial Concentration", MBC). In der MBC wird geprüft, ob nur eine Wachstumshemmung der Bakterien vorliegt, oder die Bakterien auch tatsächlich getötet werden. Der MBC Wert ist die Konzentration, bei der gerade noch 99,9% der Bakterien getötet werden. Bevorzugt weist ein Polyguanidin einen MBC-Wert von weniger als 100 µg/mL, stärker bevorzugt einen MBC-Wert von weniger als 50 µg/mL, insbesondere einen MBC-Wert von weniger als 25 µg/mL auf.

Wie oben beschrieben enthält die Polymerkomponente A neben dem als Komponente A1 eingesetzten Polyguanidin noch ein Polymer enthaltend mindestens eine funktionelle Gruppe, welche mit A1 reagieren kann als Polymer A2. Die Menge an Polymer A2 kann hierbei beliebig sein, sofern noch Polyguanidin in der erfindungsgemäßen Menge vorhanden ist.

Gemäß einer bevorzugten Ausführungsform wird das (thermoplastische) Polymer A2 in einer Menge von 85 bis 99,8 Gew.-%, bevorzugt in einer Menge von 90 bis 99,8 Gew.-%, bezogen auf die Gesamtmasse an Polymerkomponente A, eingesetzt.

Das eingesetzte Polymer A2 kann hierbei einen beliebigen Anteil an mit Komponente A1 reagierenden funktionellen Gruppen (etwa als Anhydrid, z.B. als Maleinsäureanhydrid) enthalten. Gemäß einer bevorzugten Ausführungsform weist das Polymer A2 einen Anteil an Maleinsäureanhydrid von 0,1 bis 10 Gew.-%, bezogen auf die die Gesamtmenge des Polymers A2, auf.

Gemäß einer stark bevorzugten Ausführungsform wird das Polymer A2 in einer Menge von 90 bis 99,8 Gew.-%, bezogen auf die Gesamtmasse an Polymerkomponente A, eingesetzt, und weist A2 einen Anteil an die funktionelle (daher mit Komponente A1 reagierende) Komponente tragenden Monomeren von 0,1 bis 10 Gew.-%, insbesondere 1 bis 5 Gew.-%, bezogen auf die die Gesamtmenge des Polymers A2, auf.

Bevorzugt besteht das Polymer A2 aus:
0,1 bis 10 Gew.-% mit A1 reagierender Monomere;
4,9 bis 95 Gew.-%, insbesondere 50 bis 95 Gew.-%, Styrol und
5 bis 94,9 Gew.-%, insbesondere 5 bis 49,9 Gew.-%, Acrylnitril.
Noch stärker bevorzugt besteht das Polymer A2 aus:
0,1 bis 10 Gew.-% Maleinsäureanhydrid;
4,9 bis 95 Gew.-%, insbesondere 50 bis 95 Gew.-%, Styrol und
5 bis 94,9 Gew.-%, insbesondere 5 bis 49,9 Gew.-%, Acrylnitril.

Gemäß einer stärker bevorzugten Ausführungsform weist das Polymer A2 einen Anteil an Maleinsäureanhydrid von 1 bis 5 Gew.-%, bezogen auf die die Gesamtmenge des Polymers A2, auf.

Stärker bevorzugt besteht das Polymer A2 aus:
1 bis 5 Gew.-% Maleinsäureanhydrid;
5 bis 94 Gew.-%, insbesondere 50 bis 95 Gew.-%, Styrol und
5 bis 94 Gew.-%, insbesondere 5 bis 49 Gew.-%, Acrylnitril.

Ganz besonders bevorzugt besteht das Polymer A2 aus:
1 bis 5 Gew.-% Maleinsäureanhydrid;
69 bis 80 Gew.-% Styrol und
19 bis 30 Gew.-% Acrylnitril.

Gemäß einer noch bevorzugten Ausführungsform wird das als Polymer A2 eingesetzte Styrol-Acrylnitril-Maleinsäureanhydrid-Terpolymer in einer Menge von 90 bis 99,8 Gew.-%, bezogen auf die Gesamtmasse an Polymerkomponente A, eingesetzt, und A2 weist einen Anteil an Maleinsäureanhydrid von 0,1 bis 10 Gew.-%, insbesondere 1 bis 5 Gew.-%, bezogen auf die die Gesamtmenge des Polymers A2, auf.

Daher besteht die antimikrobiell wirksame Polymerkomponente A bevorzugt aus:
90 bis 99,8 Gew.-%, insbesondere 95 bis 99 Gew.-% Polymer A2 und
0,2 bis 10 Gew.-%, , insbesondere 1 bis 5 Gew.-%, Polyguanidin als Polymer A1,
wobei die Gew.-% jeweils auf die Gesamtmasse an Komponente A bezogen sind.

Die antimikrobiell wirksame Polymerkomponente A besteht bevorzugt aus:
90 bis 99,8 Gew.-% Styrol-Acrylnitril-Maleinsäureanhydrid-Terpolymer (SAN MA) als Polymer A2 und 0,2 bis 10 Gew.-% Polyguanidin als Komponente A1,wobei die Gew.-% jeweils auf die Gesamtmasse an Polymerkomponente A bezogen sind.

Stärker bevorzugt besteht die antimikrobiell wirksame Polymerkomponente A aus:
95 bis 99 Gew.-% Styrol-Acrylnitril-Maleinsäureanhydrid-Terpolymer (SAN MA) als Polymer A2 und 1 bis 5 Gew.-% Polyguanidin als Komponente A1, wobei die Gew.-% jeweils auf die Gesamtmasse an Polymerkomponente A bezogen sind.

Besonders bevorzugt besteht die thermoplastische Formmasse M aus:
(A) 2 bis 80 Gew.-% (beispielsweise 2 bis 79,8 Gew.-%) einer antimikrobiell wirksamen Polymerkomponente A, bestehend aus
   (A1) 0,2 bis 10 Gew.-%, insbesondere 1 bis 5 Gew.-%, bezogen auf die Gesamtmasse an Polymerkomponente A, an Polyguanidin als Komponente A1 und
   (A2) 90 bis 99,8 Gew.-%, insbesondere 95 bis 99 Gew.-%, bezogen auf die Gesamtmasse an Polymerkomponente A, an Polymer A2 (insbesondere Styrol-Acrylnitril-Maleinsäureanhydrid-Terpolymer (SAN MA) als Polymer A2), wobei der Anteil an Maleinsäureanhydrid 1 bis 10 Gew.-%, bezogen auf die Gesamtmenge des Polymers A2, beträgt;
(B) 20 bis 98 Gew.-% (beispielsweise 20 bis 97,8 Gew.-%) einer weiteren thermoplastischen Polymerkomponente B und
(C) 0,2 bis 15 Gew.-% eines oder mehrerer Additive oder Hilfsmittel als Komponente C,
wobei die Summe der Gew.-% der Komponenten A, B und C zusammen 100 Gew.-% ergibt.

Die weitere thermoplastische Polymerkomponente B kann eine beliebige thermoplastische Polymerkomponente oder eine Mischung aus mehreren thermoplastischen Polymerkomponenten sein.

Die thermoplastische Formmasse M kann beliebige Mengen einer weiteren thermoplastischen Polymerkomponente B enthalten. Bevorzugt enthält die thermoplastische Formmasse M 20 bis 99 Gew.-% einer thermoplastischen Polymerkomponente B. Beispielsweise kann die thermoplastische Formmasse M 20 bis 95 Gew.-%, 20 bis 40 Gew,-%, 30 bis 50 Gew.-%, 40 bis 60 Gew.-%, 50 bis 70 Gew.-%, 50 bis 95 Gew.-%, 60 bis 80 Gew.-%, 70 bis 90 Gew.-% oder 80 bis 99 Gew.-% einer thermoplastischen Polymerkomponente B enthalten.

Gemäß einer bevorzugten Ausführungsform ist die thermoplastischen Polymerkomponente B ausgewählt aus der Gruppe bestehend aus Styrol-Copolymeren, Styrol-Butadien-Blockcopolymeren, Polycarbonaten, Polyamiden, Poly(meth)-acrylaten und Polyestern. Denkbar sind auch alternativ oder zusätzlich weitere (vorzugsweise thermoplastische) Polymere, die entsprechend der gewünschten speziellen Verwendung ausgewählt werden können.

Gemäß einer bevorzugten Ausführungsform enthält die Formmasse M mindestens 20 bis 99 Gew.-% einer thermoplastischen Polymerkomponente B ausgewählt aus der Gruppe bestehend aus Styrol-Copolymeren, Styrol-Butadien-Blockcopolymeren, Polycarbonaten, Polyamiden, Poly(meth)-acrylaten und Polyestern.

Ein Styrol-Copolymer im Sinne der Erfindung kann jedes beliebige Styrol-Copolymer sein. Besonders bevorzugt ist als Styrol-Copolymer: Acrylnitril-Butadien-Styrol-Copolymer (ABS).Gemäß einer stärker bevorzugten Ausführungsform enthält die Formmasse M 20 bis 95 Gew.-% an Styrol-Copolymer, insbesondere an Acrylnitril-Butadien-Styrol-Copolymer (ABS) als Polymerkomponente B.

Gemäß einer noch stärker bevorzugten Ausführungsform enthält die Formmasse M 50 bis 95 Gew.-% an Styrol-Copolymer, insbesondere an Acrylnitril-Butadien-Styrol-Copolymer (ABS) als Polymerkomponente B.

Gemäß einer bevorzugten Ausführungsform besteht die Formmasse M aus:
(A) 1 bis 50 Gew.-%, insbesondere 2 bis 49,8 Gew.-% einer antimikrobiell wirksamen Polymerkomponente A, enthaltend
   (A1) 0,2 bis 25 Gew.-%, bezogen auf die Gesamtmasse an Polymerkomponente A, an Polyguanidin als Komponente A1 und
   (A2) 75-99,8 Gew.-%, bezogen auf die Gesamtmasse an Polymerkomponente A, an Polymer A2;
(B) 50 bis 99 Gew.-% einer weiteren thermoplastischen Polymerkomponente B, insbesondere Acrylnitril-Butadien-Styrol-Copolymer (ABS) und
(C) 0 bis 10 Gew.-%, insbesondere 0,2 bis 10 Gew.-% eines oder mehrerer Additive oder Hilfsmittel als Komponente C,
wobei die Summe der Gew.-% der Komponenten A, B und C zusammen 100 Gew.-% ergibt.

Häufig besteht die thermoplastische Formmasse M aus:
(A) 2 bis 50 Gew.-% (beispielsweise 2 bis 49,8 Gew.-%) einer antimikrobiell wirksamen Polymerkomponente A, bestehend aus
   (A1) 1 bis 5 Gew.-%, bezogen auf die Gesamtmasse an Polymerkomponente A, an Polyguanidin als Komponente A1 und
   (A2) 95 bis 99 Gew.-%, bezogen auf die Gesamtmasse an Polymerkomponente A, an Polymer A2, wobei der Anteil an Maleinsäureanhydrid 1 bis 10 Gew.-% bezogen auf die die Gesamtmenge des Polymers A2, beträgt;
(B) 50 bis 98 Gew.-% (beispielsweise 50 bis 97,8 Gew.-%) eines oder mehrerer Stryrol-Copolymere, insbesondere Acrylnitril-Butadien-Styrol-Copolymer (ABS); und
(C) 0,2 bis 15 Gew.-% eines oder mehrerer Additive oder Hilfsmittel als Komponente C,
wobei die Summe der Gew.-% der Komponenten A, B und C zusammen 100 Gew.-% ergibt.

Gemäß einer bevorzugten Ausführungsform besteht die Formmasse M aus:
(A) 1 bis 50 Gew.-%, insbesondere 2 bis 49,8 Gew.-% einer antimikrobiell wirksamen Polymerkomponente A, enthaltend
   (A1) 0,2 bis 25 Gew.-%, bezogen auf die Gesamtmasse an Polymerkomponente A, an Polyguanidin als Komponente A1 und
   (A2) 75-99,8 Gew.-%, bezogen auf die Gesamtmasse an Polymerkomponente A, an Styrol-Acrylnitril-Maleinsäureanhydrid-Terpolymer als Polymer A2;
(B) 50 bis 99 Gew.-% einer weiteren thermoplastischen Polymerkomponente B, insbesondere Acrylnitril-Butadien-Styrol-Copolymer (ABS) und
(C) 0 bis 10 Gew.-%, insbesondere 0,2 bis 10 Gew.-% eines oder mehrerer Additive oder Hilfsmittel als Komponente C,
wobei die Summe der Gew.-% der Komponenten A, B und C zusammen 100 Gew.-% ergibt.

Besonders bevorzugt besteht die thermoplastische Formmasse M aus:
(A) 2 bis 50 Gew.-% (beispielsweise 2 bis 49,8 Gew.-%) einer antimikrobiell wirksamen Polymerkomponente A, bestehend aus
   (A1) 1 bis 5 Gew.-%, bezogen auf die Gesamtmasse an Polymerkomponente A, an Polyguanidin als Komponente A1 und
   (A2) 95 bis 99 Gew.-%, bezogen auf die Gesamtmasse an Polymerkomponente A, an Styrol-Acrylnitril-Maleinsäureanhydrid-Terpolymer (SAN MA) als Polymer A2, wobei der Anteil an Maleinsäureanhydrid 1 bis 10 Gew.-% bezogen auf die die Gesamtmenge des Piolymers A2, beträgt;
(B) 50 bis 98 Gew.-% eines oder mehrerer Stryrol-Copolymere, insbesondere Acrylnitril-Butadien-Styrol-Copolymer (ABS); und
(C) 0,2 bis 15 Gew.-% eines oder mehrerer Additive oder Hilfsmittel als Komponente C,
wobei die Summe der Gew.-% der Komponenten A, B und C zusammen 100 Gew.-% ergibt.

Die eingesetzten Polymerkomponenten A und B können kristallin oder amorph sein. Gemäß einer bevorzugten Ausführungsform sind mindestens 50 Gew.-% der in der Formmasse M enthaltenen polymeren Komponenten amorphe Polymere. Bevorzugt sind mindestens 60 Gew.-%, stärker bevorzugt mindestens 70 Gew.-%, noch stärker bevorzugt mindestens 80 Gew.-%, noch stärker bevorzugt mindestens 90 Gew.-%, insbesondere mindestens 95 Gew.-% der in der Formmasse M enthaltenen polymeren Komponenten amorphe Polymere.

Als Additive oder Hilfsstoffe C können der thermoplastischen Formmasse M beliebige im Stand der Technik bekannte Polymeradditive zugesetzt werden. Beispielshaft kann ein Additiv oder ein Hilfsstoff ausgewählt sein aus der Gruppe bestehend aus Antioxidantien, UV-Stabilisatoren, Peroxid-Zerstörer, Antistatika, Gleitmitteln, Entformungsmitteln, Flammschutzmitteln, Füll- oder Verstärkerstoffen (Glasfasern, Kohlefasern, etc.), Farbmitteln und Kombinationen aus zwei oder mehr daraus.

Die vorliegende Erfindung betrifft auch die Herstellung einer antimikrobiell wirksamen thermoplastischen Formmasse M. Daher betrifft die vorliegende Erfindung ein Verfahren zur Herstellung einer antimikrobiell wirksamen thermoplastischen Formmasse M enthaltend die folgenden Verfahrensschritte:
(i) Bereitstellen der folgenden Komponenten:
   (A1) Polyguanidin als Komponente A1 und
   (A2) Polymer A2, enthaltend mindestens eine funktionelle Gruppe, welche mit A1 reagieren kann; und optional

   (B) weiterer thermoplastischer Komponente(n) B und optional
   (C) eines oder mehrerer Additive oder Hilfsmittel als Komponente C;
(ii) Extrudieren einer Mischung der Komponenten A1 und A2 und optional C zu einer antimikrobiell wirksamen, thermoplastischen Polymerkomponente A und
(iii) Extrudieren der aus Schritt (ii) erhaltenen antimikrobiell wirksamen Polymerkomponente A mit einer oder mehreren weiteren thermoplastischen Polymerkomponenten B und/oder einem oder mehreren Additiven oder Hilfsmitteln als Komponente C, wobei erfindungsgemäß während dem Schritt (iii) Polyguanidin als Komponente A1 zugesetzt wird.

Gemäß einer bevorzugten Ausführungsform betrifft die vorliegende Erfindung ein Verfahren zur Herstellung einer antimikrobiell wirksamen thermoplastischen Formmasse M enthaltend die folgenden Verfahrensschritte:
(i) Bereitstellen der folgenden Komponenten:
   (A1) Polyguanidin als Komponente A1 und
   (A2) Styrol-Acrylnitril-Maleinsäureanhydrid-Terpolymer als Polymer A2; und optional
   (B) weiterer thermoplastischer Komponente(n) B und optional
   (C) eines oder mehrerer Additive oder Hilfsmittel als Komponente C;
(ii) Extrudieren einer Mischung der Komponenten A1 und A2 und optional C zu einer antimikrobiell wirksamen, thermoplastischen Polymerkomponente A und
(iii) Extrudieren der aus Schritt (ii) erhaltenen antimikrobiell wirksamen Polymerkomponente A mit einer oder mehreren weiteren thermoplastischen Polymerkomponenten B und/oder einem oder mehreren Additiven oder Hilfsmitteln als Komponente C, wobei erfindungsgemäß während dem Schritt (iii) Polyguanidin als Komponente A1 zugesetzt wird.

Hierbei gelten die gleichen Definitionen und bevorzugten Ausführungsformen wie oben bezüglich der thermoplastischen Formmasse M beschrieben. Daher ist gemäß einer bevorzugten Ausführungsform die thermoplastische Formmasse eine thermoplastische Formmasse M gemäß der vorliegenden Erfindung wie oben beschrieben.

Alle Komponenten (A1, A2, B und C) können kommerziell erhalten werden. Alternativ können einzelne, mehrere oder alle Komponenten A1, A2, B und/oder C optional auch im Sinne der vorliegenden Erfindung hergestellt werden. Für das Verfahren wird das erfindungsgemäß einzusetzende Polyguanidin bevorzugt als Feststoff mit der Komponente A2 (insbesondere den Styrol-Acrylnitril-Maleinsäureanhydrid-Terpolymeren) und ggf. weiteren Polymeren auf Acrylnitril-Styrol-Basis compoundiert und muss bevorzugt nicht als Lösung zugegeben werden.

Beispielsweise kann das als Komponente A1 verwendete Polyguanidin durch Reaktion von Guanidinhydrochlorid (GHC) mit 1,6-Hexamethylendiamin (HMDA) erhalten werden. Hierzu kann das HMDA geschmolzen werden (etwa bei einer Temperatur von 70 bis 80°C) und dem geschmolzenen HMDA kann nach und nach GHC unter Rühren zugesetzt werden (etwa bei einer Temperatur von 100 bis 200°C). Hierbei kann ein beliebiges HMDA: GHC-Verhältnis eingesetzt werden. Beispielhaft kann ein HMDA: GHC-Verhältnis von 1:2 bis 2:1, bevorzugt von 1:1 bis 1:1,5, beispielsweise von 0,9:1,1, eingesetzt werden. Nach Abschluss der Reaktion nach mehreren Stunden, beispielsweise 4 bis 6 h Rühren, kann der Reaktionsansatz mit Wasser versetzt werden.
Hierbei kann beispielsweise eine 60 bis 70 gew.-%ige wässrige Polyguanidin-Lösung erhalten werden. Es kann optional wie im unten ausgeführten Beispielteil verfahren werden.

Wird in der thermoplastischen Formmasse M neben Polymer A2 (z.B. PG/SAN MA) noch mindestens eine weitere thermoplastische Polymerkomponente B eingesetzt, kann Polymer A2 als A2-Masterbatch eingesetzt werden. Das bedeutet, falls neben Polymer A2 noch mindestens eine weitere thermoplastischen Polymerkomponente B eingesetzt wird, optional erst Polymer A2 als Polymerkomponente A hergestellt werden kann und hernach mit einer oder mehreren thermoplastischen Polymerkomponenten B compoundiert wird.

Compoundiert ist wie hierin verwendet im weitesten Sinne zu verstehen als jede Vermischung zu einer thermoplastischen Formmasse M, insbesondere durch Extrusion. Der Fachmann wird erkennen, dass die Compoundierungs-Bedingungen, wie z.B. die Compoundierungs-Temperatur, von den jeweiligen Komponenten, etwa der weiteren thermoplastischen Polymerkomponente B, abhängen.

So kann beispielsweise eine Compoundierungs-Temperatur von 200 bis 250°C verwendet werden. Beispiele sind im unten stehende experimentellen Beispielsteil erläutert.

Erfindungsgemäß enthält die thermoplastische Formmasse M auch Komponente B, wird auch Schritt (iii) (des Extrudierens der aus Schritt (ii) erhaltenen antimikrobiell wirksamen Polymerkomponente A mit einer oder mehreren weiteren thermoplastischen Polymerkomponenten B und/oder einem oder mehreren Additiven oder Hilfsmitteln als Komponente C) durchgeführt und wird während dem Schritt (iii) Polyguanidin als Komponente A1 zugesetzt.

Dies kann besonders hohe Beladungen mit Polyguanidin A1 in der thermoplastischen Formmasse M auch bei vergleichsweise niedrigen Gehalten an Komponente A2 ergeben. Zudem ist das Ausbluten vergleichsweise gering.

Überraschend wurde zudem gefunden, dass es auch bei höheren Gehalten an Polyguanidin A1 in der thermoplastischen Formmasse M nicht zu nennenswerter, unerwünschter Vernetzung der Polymermatrix kam. Würde man dagegen gleich bei der Herstellung eines A1/A2-Masterbatches mit hohen Gehalten an A1 arbeiten, wäre eine unerwünschte Vernetzung der Polymermatrix zu erwarten.

So kann etwa eine Lösung, die das Polyguanidin A1 enthält, während des Extrudierens (daher währen der Durchführung des Schritts (iii)) zudosiert werden. Beispielhaft kann das Zudosieren von Polyguanidin A1 (beispielsweise PHMG) während des Extrudierens zwischen 10 und 100 g pro kg thermoplastischer Formmasse M betragen. Bevorzugt beträgt das Zudosieren von Polyguanidin an A1 während des Extrudierens zwischen 20 und 60 g an A1 pro kg thermoplastischer Formmasse M, insbesondere zwischen 30 und 50 g an A1 pro kg thermoplastischer Formmasse M.

Es kann beispielsweise für jeweils 1 Gew-% zusätzliches Polyguanidin A1 (etwa PHMG) pro 1 kg/h Materialdurchsatz (ca.) 10-12 g/h Polyguanidin A1 (etwa PHMG) eingesetzt werden. Andere Materialdurchsätze und gewünschte Massenanteile können leicht, etwa mittels eines einfachen Dreisatzes, umgerechnet werden. Daher kann beispielsweise für jeweils 1 Gew-% zusätzliches Polyguanidin A1 (etwa PHMG) (bei einem Materialdurchsatz von beispielsweise 4 kg/h) ca. 40-50 g/h Polyguanidin A1 (etwa PHMG) eingesetzt werden.

So können beispielhaft für 3-5 Gew-% zusätzliches PHMG bei einem angestrebten Materialdurchsatz von ca. 3-5 kg/h ca. 125-210 g/h PG bzw. ca. 155-260 g/h Polyguanidin- (PG-)Lösung (-70 Gew-% PG) eingesetzt werden. So können beispielhaft für etwa 4 Gew-% zusätzliches PHMG bei einem angestrebten Materialdurchsatz von ca. 4 kg/h ca. 167g/h PG bzw. ca. 207 g/h PG Lösung (-70 Gew-% PG) eingesetzt werden.

Gemäß einer weiteren bevorzugten Ausführungsform wird während dem Schritt (ii) (des Extrudierens einer Mischung der Komponenten A1 und A2 und optional C) Polyguanidin als Komponente A1 zugesetzt. Gemäß einer weiteren bevorzugten Ausführungsform wird während der Schritte (ii) und (iii) jeweils Polyguanidin als Komponente A1 zugesetzt.

Bei der Zugabe kann die Polyguanidin-Lösung optional vorgewärmt werden, etwa auf 30-65°C, bevorzugt 40-60°C, bevorzugt 45-55°C, insbesondere (etwa) 50°C.

Die Bereitstellung einer erfindungsgemäßen thermoplastischen Formmasse M (etwa als Kunststoffgranulat zur weiteren Verarbeitung) ist in vielerlei Hinsicht vorteilhaft. So ist ein Kunststoffgranulat nicht nur gut lagerbar, sondern auch gut dosierbar und entsprechend schnell und einfach zu verarbeiten, so etwa zu beliebigen Formteilen F und/oder Produkten. Es wird daher verstanden werden, dass aus der erfindungsgemäßen thermoplastischen Formmasse M beliebige Formteile F hergestellt werden können.

Daher betrifft ein weiterer Aspekt der vorliegenden Erfindung ein Formteil F, enthaltend mindestens eine thermoplastische Formmasse M gemäß der vorliegenden Erfindung oder mindestens eine thermoplastische Formmasse M erhältlich nach einem Verfahren gemäß der vorliegenden Erfindung. Ein solches erfindungsgemäßes Formteil F kann aus der erfindungsgemäßen thermoplastischen Formmasse M auf beliebige Weise hergestellt werden. Dem Fachmann sind zahlreiche Verfahren bekannt, aus thermoplastischen Formmassen Formteile herzustellen. Gemäß einer bevorzugten Ausführungsform ist das Formteil F hergestellt durch Extrudieren, Spritzgießen, Blasformen, Verspinnen oder einen Sprühvorgang.

Entsprechend kann das Formteil F beispielsweise ein ausgefülltes Formteil F sein, das (weitgehend) aus der ausgehärteten thermoplastischen Formmasse M besteht. Alternativ kann das Formteil F ein Hohlkörper sein, der (weitgehend) aus der ausgehärteten thermoplastischen Formmasse M besteht. Alternativ kann das Formteil F eine Folie sein, die (weitgehend) aus der ausgehärteten thermoplastischen Formmasse M besteht. Alternativ kann das Formteil F ein(e)(weitgehend) aus der ausgehärteten thermoplastischen Formmasse M bestehende(r) Faden, Faser oder Faserbündel sein. Alternativ kann das Formteil F eine (weitgehend) aus der ausgehärteten thermoplastischen Formmasse M bestehende Beschichtung sein.

Optional kann das Formteil F selbst ein Produkt oder ein Halbzeug darstellen oder kann ein Zwischenprodukt sein, das noch weiterverarbeitet wird. Es wird verstanden werden, dass ein Formteil F optional beispielsweise lackiert, besprüht, beschichtet, bedruckt oder anderweitig weiterverarbeitet werden kann. Auch kann ein Produkt optional aus mehreren erfindungsgemäßen Formteilen F zusammengesetzt sein.

Gemäß einem weiteren Aspekt betrifft die vorliegende Erfindung daher ein Produkt bestehend aus oder enthaltend mindestens ein(em) Formteil F gemäß der vorliegenden Erfindung.

Ein Produkt im Sinne der vorliegenden Erfindung kann ein beliebiges Produkt sein. Beispielsweise kann das Produkt ein Kunststoffgegenstand sein. Das Produkt kann dann die durch den enthaltenen Wirkstoff eine entsprechende antimikrobielle Wirkung haben. Solche Produkte können in vielen Bereichen des täglichen Lebens vorteilhaft sein.

Gemäß einer bevorzugten Ausführungsform ist das Produkt ausgewählt aus der Gruppe von: Fahr- und Flugzeugteilen, Schiffsteilen, Verpackungen, Rohren, Schläuchen, Sanitärartikeln, Medizinprodukten, Eingabegeräten und Bedienelementen, Laborgeräten- und Verbrauchsgütern, Maschinenteilen, Haushaltsgeräten, Mobiliar, Griffen, Dichtungen, Bodenbelägen, Textilien, Agrargeräten, Schuhsohlen, Gefäßen zur Aufbewahrung von Nahrungs- und Futtermitteln, Geschirr, Besteck, Filtern, Fernsprechgeräten, und Beschichtungen.

Optional können alternativ auch Chromatographie-Matrices und/oder Mikro- und/oder Nanopartikeln ein Produkt im Sinne der vorliegenden Erfindung sein.

Rohre können beispielsweise (Ab)Wasserrohre sein. Medizinprodukte können beispielsweise Gebrauchsgegenstände im Medizinbereich sein. Sanitärartikel könne beispielsweise Duschvorhänge oder Toilettenteile (z.B. Toilettenbrillen) sein. Agrargeräte können auch Futtermittelanlagen sein. Zu Geschirr können beispielsweise Teller und Trinkgefäße gehören. Zu Eingabegeräten und Bedienelementen können beispielsweise auch Tastaturen gehören. Beschichtungen können beispielsweise auch antibakterielle Anstriche sein. Eine Vielzahl weiterer Gegenstände ist selbstverständlich vorstellbar. Die thermoplastische Formmasse M gemäß der vorliegenden Erfindung kann in beliebiger Weise verwendet werden. Insbesondere kann die thermoplastische Formmasse M zur Verhinderung von mikrobiellem Wachstum eingesetzt werden.

Gemäß einem weiteren Aspekt betrifft die vorliegende Erfindung die Verwendung einer thermoplastischen Formmasse M gemäß der vorliegenden Erfindung oder einer Formmasse M erhältlich nach einem Verfahren gemäß der vorliegenden Erfindung zur Herstellung eines antimikrobiellen Formteils F zur Verhinderung von mikrobiellem Wachstum. Entsprechend betrifft die vorliegende Erfindung auch die Verwendung eines Formteils F der vorliegenden Erfindung und/oder eines Produkts gemäß der vorliegenden Erfindung zur Verhinderung von mikrobiellem Wachstum. So kann beispielsweise ein Produkt selbst oder eine Beschichtung hergestellt werden, die vermindertes mikrobielles Wachstum zeigen.

Gemäß einer bevorzugten Ausführungsform ist die Verwendung die Verhinderung der Ausbildung eines Bio-Films auf der Oberfläche von oder in dem Formteil F.

So kann beispielsweise eine sauberere Oberfläche in Bereichen hergestellt werden, in denen eine Oberfläche Flüssigkeiten und/oder (Luft)feuchtigkeit ausgesetzt ist, beispielsweise bei Produkten wie oben beschrieben, bei denen optisch gute Eigenschaften, Hygiene und/oder Haltbarkeit des Produkts bestehend aus der oder umfassend die thermoplastische(n) Formmasse M entscheidend sind.

Entsprechend betrifft ein weiterer Aspekt der vorliegenden Erfindung ein Verfahren zur Verhinderung von mikrobiellem Wachstum umfassend die folgenden Schritte:
(i) Bereitstellen einer thermoplastischen Formmasse M gemäß der vorliegenden Erfindung oder Formmasse M erhältlich nach einem Verfahren gemäß der vorliegenden Erfindung und
(ii) Herstellen des Formteils F, insbesondere durch Extrudieren, Spritzgießen, Blasformen, Verspinnen oder einen Sprühvorgang.

Gemäß einer bevorzugten Ausführungsform ist das Verfahren geeignet zur Verhinderung der Ausbildung eines Bio-Films auf der Oberfläche von oder in dem Formteil F.

Die Erfindung wird weiter durch die unten ausgeführten experimentellen Beispiele und die Ansprüche erläutert.

### Synthese des Polyquanidins

Das hier eingesetzte Polyguanidin wird aus Guanidinhydrochlorid (GHC) und 1,6 Hexamethylendiamin (HMDA) synthetisiert. Chemikalien: Guanidinhydrochlorid, GHC (CAS: 50-01-1), Bezugsquelle: ACROS Organics; 1,6 Hexamethylendiamin (HMDA) (CAS: 124-09-4), Bezugsquelle: NIGU AlzChem.

Vor Reaktionsbeginn wird das per Destillation (130 °C, 40 mbar) aufgereinigte HMDA bei 60 °C im Trockenschrank aufgeschmolzen. GHC wird wie erhalten eingesetzt. Ein ausgeheizter 4L-Dreihalskolben wird mit KPG-Rührer, zwei Kühlern übereinander (1. Schlangenkühler, 2. Intensivkühler) und Innenthermometer bestückt. Ein unter dem Kolben platziertes Ölbad ist auf 60 °C vorgeheizt. Der KPG-Rührer wird auf 300 U/min eingestellt. Nacheinander werden 374,9 g GHC (3,92 mol, 1,1äq) und 116,21 g HMDA (3,57 mol, 0,9 äq) eingewogen. Die Reaktion wird mit folgendem Temperaturprofil durchgeführt (Ölbadtemperatur): 1h Rühren bei 110°C, 4h Rühren bei 190°C und 1h Rühren bei 190°C und 250mbar. Am Ende der Reaktion wird aus der Schmelze eine Probe gezogen, anschließend die Heizquelle entfernt, vorsichtig auf Raumtemperatur heruntergekühlt und anschließend 284,3 g dest. Wasser zum Auflösen eingefüllt, so dass eine ca. 70 gew.-%ige Lösung entsteht. Von dieser Probe werden Tests auf antimikrobielle Wirksamkeit durchgeführt (MIC, MBC). Die Zusammensetzung des Polyguanidins wird mittels Elementaranalyse verifiziert.

Das Molekulargewicht wird mittels Gel-Permeations-Chromatographie (GPC) bestimmt. Im vorliegenden Beispiel beläuft sich das Molekulargewicht auf Mₙ = 5500, gemessen in Hexafluorisopropanol gegen PMMA-Standards.

### Erfindungsgemäße Beispiele

Das zuvor synthetisierte Polyguanidin (PG) wird mit Styrol-Acrylnitril-Maleinsäureanhydrid-Terpolymer (SAN MA) VT2421 (Hersteller: INEOS Styrolution), Maleinsäureanhydridgehalt 2,1 Gew-%, extrudiert. Es werden drei Masterbatches mit 5, 10 und 15 Gew-% PG-Gehalt in SAN MA auf einem Doppelschneckenextruder DSE 20/40 mit Plasti-Corder der Firma Brabender hergestellt (Proben A1 bis A3).

Bei der Herstellung des Masterbatches mit 15 Gew-% PG zeigt sich ein sofortiger starker Druckanstieg bei Zugabe des Polyguanidins zur Polymerschmelze. Der extrudierte Strang ist inhomogen und spröde, so dass für die folgenden Compoundier-Versuche nur der 5 gew-%ige und 10 gew-%ige PG/SAN MA-Masterbatch weiter betrachtet werden.

Der so erhaltene 5 gew-%ige und 10 gew-%ige PG/SAN MA-Masterbatch (Probe A1 und Probe A2) werden im Folgenden mit SAN MA und mit Terluran® GP-22 (INEOS Styrolution), einem leicht fließenden, schlagzähen Acrylnitril-Butadien-Styrolpolymer (ABS), compoundiert, um Compounds mit geringeren Gehalten an Polyguanidin herzustellen. Die Compoundierung wird auf einem DSM Micro 15cc Twin Screw Compounder der Firma Xplore durchgeführt.

**Tabelle 1: Hergestellte Compounds (Komponenten) für erfindungsgemäße Beispiele**

| Probe | Polymer-Master terbatch | PG-Gehalt im Polymer-Masterbatch [Gew-%] | Polymer für Verdünnung | Verdünnung (Masterbatch: Verdünnung) | Polyguanidin-Gehalt [Gew-%] | Compoundierungs-Temperatur [°C] |
|---|---|---|---|---|---|---|
| A1 | A1 | 5 | - | - | 5 | |
| A1-1 | A1 | 5 | SAN MA | 1:5 | 1 | 210 |
| A1-2 | A1 | 5 | Terluran® GP-22 | 1:5 | 1 | 230 |
| A1-3 | A1 | 5 | Terluran® GP-22 | 1:5 | 1 | 250 |
| A2 | A1 | 10 | - | - | 10 | |
| A2-1 | A1 | 10 | SAN MA | 1:10 | 1 | 210 |
| A2-2 | A1 | 10 | SAN MA | 1:2 | 5 | 210 |
| A2-3 | A1 | 10 | Terluran® GP-22 | 1:2 | 5 | 230 |
| A2-4 | A1 | 10 | Terluran® GP-22 | 1:10 | 1 | 230 |
| A3 | A3 | 15 | - | - | 15 | |

Die so dargestellten Masterbatche und Compounds werden auf antimikrobielle Wirksamkeit (Schüttelflaschentest, Kirby-Bauer-Test) und auf Ausbluten getestet. Außerdem wird die Schmelze-Volumenfließrate bestimmt (MVR).

### Vergleichsbeispiele: Polyguanidin-ABS-Compounds

Das zuvor synthetisierte Polyguanidin (PG) wird mit Terluran® GP-22 (Handelsprodukt der INEOS Styrolution, Frankfurt), einem leicht fließenden, schlagzähen Acrylnitril-Butadien-Styrolpolymer (ABS), in einem Scientific Prozess 11 Doppelschneckenextruder der Firma Thermo Fischer extrudiert. Es werden ein 15 gew.-%iger PG/ABS-Masterbatch (Probe B1) sowie ein 25 gew-%iger PG/ABS-Masterbatch (Probe B2) hergestellt. Die erhaltenen Granulate zeigen eine homogene weiße Farbgebung.

Der so erhaltene 15 gew.-%ige PG/ABS-Masterbatch wird im Folgenden wiederum mit Terluran® GP-22 (ABS von INEOS Styrolution) compoundiert, um Compounds mit geringeren Gehalten an Polyguanidin herzustellen. Die Compoundierung wird mit einem DSM Micro 15cc Twin Screw Compounder (der Firma Xplore) durchgeführt.

**Tabelle 2: Hergestellte Compounds für Vergleichsbeispiele**

| Probe | Polymer-Master terbatch | PG-Gehalt im Polymer-Masterbatch [Gew-%] | Polymer für Verdünnung | Verdünnung (Masterbatch : Verdünnung) | Polyguanidin-Gehalt [Gew-%] | Compoundierungs-Temperatur [°C] |
|---|---|---|---|---|---|---|
| B1 | B1 | 15 | - | - | 15 | |
| B1-1 | B1 | 15 | Terluran® GP-22 | 1:15 | 1 | 220 |
| B1-2 | B1 | 15 | Terluran® GP-22 | 1:3 | 5 | 220 |
| B1-3 | B1 | 15 | Terluran® GP-22 | 2:3 | 10 | 220 |
| B2 | B2 | 25 | - | - | 25 | |

Die so dargestellten Masterbatche und Compounds werden auf antimikrobielle Wirksamkeit (Schüttelflaschentest, Kirby-Bauer-Test) und auf Ausbluten getestet. Außerdem wird die Schmelze-Volumenfließrate bestimmt (MVR).

### Durchführung Test "Minimum Inhibitory Concentration" (MIC)

Zur Bestimmung der minimalen Hemmkonzentration wird aus einer angesetzten Nährlösung und der Probelösung eine Verdünnungsreihe in Doppelbestimmung auf eine Mikrotiterplatte pipettiert und anschließend über Nacht bei 37 °C inkubiert. Eine Trübung der Lösung zeigt Bakterienwachstum an. Die minimale Hemmkonzentration ist der niedrigste Wert der Verdünnungsreihe, bei dem die Lösung klar geblieben ist.

### Durchführung Test zu minimaler Bakterienkonzentration

### ("Minimum Bacterial Concentration", MBC)

In diesem Test wird geprüft, ob nur eine Wachstumshemmung der Bakterien vorliegt, oder die Bakterien wirklich getötet werden. Hierzu wird aus dem letzten trüben und den ersten drei klaren Näpfchen des MIC-Tests Proben entnommen und für 24h bei 37 °C auf Agarplatten ausgestrichen inkubiert. Der MBC-Wert ist die Konzentration, bei der gerade noch 99,9% der Bakterien getötet worden sind.

### Durchführung Schüttelflaschentest

Der Test wird gemäß "Standard Test Method for determining the antimicrobial activity of immobilized antimicrobial Agents under dynamic Contact Conditions, Designation: E2149-10"; ASTM International; Beuth Verlag, durchgeführt.

Die zu untersuchenden Proben werden mit einer Arbeitslösung (Bakteriensuspension mit bekannter CFU (colony forming unit), hier Escherichia coli (E. coli), DSM-Nr. 1077, Bezugsquelle DMSZ Braunschweig) versetzt und bei 20 °C und einer Schüttelgeschwindigkeit von 200 Umdrehungen/Minute geschüttelt. Zu Beginn und nach 1 h, 3 h, 6 h und 24 h werden definierte Mengen der ursprünglichen Arbeitslösung, der zu bestimmenden Proben und der Referenz entnommen und auf einer Agarplatte ausgestrichen. Die Anzahl der Bakterienkolonien wird ausgezählt. Als Referenz dient eine mit einem ausblutenden Wirkstoff antimikrobiell ausgerüstete, nicht erfindungsgemäße Probe, deren Wirksamkeit bekannt ist. Als Negativprobe dient die angesetzte Arbeitslösung (Bakteriensuspension) ohne antibakteriell wirksamen Zusatz.

Zur Herstellung der Proben werden die aus der Schüttelflasche nach 0 h, 1 h, 3 h und 6h entnommenen Mengen halbiert. Ein Teil wird direkt auf einer Agarplatte ausgestrichen, der andere Teil wird nach 1:10 Verdünnung auf einer Agarplatte ausgestrichen. Die Verdünnung dient der besseren optischen Auswertung (Auszählen der Kolonien) des Tests, falls sehr viele Kolonien auf der Agarplatte gewachsen sind. Diese Ausstriche werden bei einer Temperatur von 37 °C inkubiert. Nach 1 h, 3 h und 6 h werden die Kolonien ausgezählt. Die Proben werden anschließend miteinander verglichen.

### Durchführung Kirby-Bauer-Test

Auf einer hydraulischen Presse werden von den erhaltenen Masterbatches und Compounds bei 210 °C und 8 kN Tabletten gepresst. Anschließend werden die gepressten Proben auf eine Agarplatte gelegt, die zuvor mit einer Bakteriensuspension von E. coli bekannter Konzentration (DSM-Nr. 1077, Bezugsquelle DSMZ Braunschweig) inokuliert und 18 h bei 37 °C inkubiert worden ist.

Die getesteten Proben werden von der Agarplatte abgehoben, ein Ösenabstrich der Fläche unterhalb der Probenkörper entnommen und auf einer neuen Agarplatte ausgestrichen. Dieser Ausstrich soll zeigen, ob die Bakterien unterhalb der Probenkörper vollständig abgetötet worden sind. Ein Hemmhof um die Probenkörper während des Tests lässt auf ein Ausbluten schließen.

### Test auf Ausbluten

Um auftretendes Ausbluten (engl. "Leaching") zu messen, werden 1 g der getrockneten Probe in einen zuvor tarierten 100 ml Rundkolben eingewogen und mit 25 ml deionisiertem Wasser bzw. Isopropanol 24 h bei Zimmertemperatur (25 °C) extrahiert. Das abfiltrierte Granulat wird getrocknet und zurückgewogen.

### Durchführung Schmelze-Volumenfließrate-Bestimmung (MVR)

Die MVR-Messungen werden mit einem Meltflixer 2000 der Firma Thermo Haake nach der Norm DIN EN ISO 1133 (Version 1:2012) durchgeführt. In das vortemperierte Gerät werden 4 - 6 g der Probe eingewogen, Stempel und Messstreifen eingesetzt, und die Probe vier Minuten auf die gewünschte Temperatur vorgeheizt (ABS = 220°C). Anschließend wird der Stempel mit einem Gewicht beschwert (ABS = 10,00 kg), und die Schmelze-Volumenfließrate (MVR [cm³/10 min]) als Mittelwert mit Standardabweichung aus sechs Messungen bestimmt.

### Test "Minimum Inhibitory Concentration" (MIC)

Die minimale Hemmkonzentration des dargestellten Polyguanidins wird wie beschrieben bestimmt. Sie beträgt 7,81 µg/mL.

### Test "Minimum Bacterial Concentration" (MBC)

Der MBC Wert (die Konzentration, bei der gerade noch 99,9% der Bakterien getötet werden) beträgt für das dargestellte Polyguanidin 15,61 µg/mL.

### Schüttelflaschentest:

**Tabelle 3: Darstellung der Ergebnisse des Schüttelflaschentests**

| | | | Anzahl der Kolonien | | | |
|---|---|---|---|---|---|---|
| Probe | PG-Gehalt | Verdünnung | zu Beginn | nach 1 h | nach 3 h | nach 6 h |
| A1 | 5 | - | 150 | 0 | 0 | 0 |
| A1 | 5 | 10% | 13 | 0 | 0 | 0 |
| A1-1 | 1 | - | 100 | 0 | 0 | 0 |
| A1-1 | 1 | 10% | 11 | 0 | 0 | 0 |
| A1-2 | 1 | - | 124 | 0 | 0 | 0 |
| A1-2 | 1 | 10% | 13 | 0 | 0 | 0 |
| A1-3 | 1 | - | 120 | 0 | 0 | 0 |
| A1-3 | 1 | 10% | 11 | 0 | 0 | 0 |
| A2 | 10 | - | 15 | 0 | 0 | 0 |
| A2 | 10 | 10% | 7 | 0 | 0 | 0 |
| A2-1 | 1 | - | 96 | 0 | 0 | 0 |
| A2-1 | 1 | 10% | 12 | 0 | 0 | 0 |
| A2-2 | 5 | - | 47 | 0 | 0 | 0 |
| A2-2 | 5 | 10% | 6 | 0 | 0 | 0 |
| A2-3 | 5 | - | 108 | 0 | 0 | 0 |
| A2-3 | 5 | 10% | 10 | 0 | 0 | 0 |
| A2-4 | 1 | - | 140 | 0 | 0 | 0 |
| A2-4 | 1 | 10% | 8 | 0 | 0 | 0 |
| A3 | 15 | - | 40 | 0 | 0 | 0 |
| A3 | 15 | 10% | 5 | 0 | 0 | 0 |
| B1 | 15 | - | 0 | 0 | 0 | 0 |
| B1 | 15 | 10% | 0 | 0 | 0 | 0 |
| B1-1 | 1 | - | 118 | 0 | 0 | 0 |
| B1-1 | 1 | 10% | 14 | 0 | 0 | 0 |
| B1-2 | 5 | - | 10 | 0 | 0 | 0 |
| B1-2 | 5 | 10% | 2 | 0 | 0 | 0 |
| B1-3 | 10 | - | 2 | 0 | 0 | 0 |
| B1-3 | 10 | 10% | 0 | 0 | 0 | 0 |
| B2 | 25 | - | 0 | 0 | 0 | 0 |
| B2 | 25 | 10% | 0 | 0 | 0 | 0 |
| Referenz | - | - | 0 | 0 | 0 | 0 |
| Referenz | - | 10% | 0 | 0 | 0 | 0 |
| Negativprobe 1 | - | - | 247 | 290 | 230 | 289 |
| Negativprobe 1 | - | 10% | 18 | 32 | 18 | 27 |
| Negativprobe 2 | - | - | 131 | 125 | 122 | 117 |
| Negativprobe 2 | - | 10% | 11 | 19 | 16 | 15 |

Alle hergestellten Proben (erfindungsgemäße Beispiele und Vergleichsbeispiele) wirken schnell antibakteriell, teils sofort, teils innerhalb einer Stunde.

### Test auf Ausbluten

Um auftretendes Ausbluten (engl. "Leaching") zu messen, werden jeweils 1 g Probe der 1:10-Verdünnung des 5 gew.-%igen SAN MA-Masterbatches (Probe A1-1) und die 1:10- und 1:1-Verdünnungen des 10 gew.-%igen SAN MA-Masterbatches (Proben A2-1 und A2-2) in einen zuvor tarierten 100 ml Rundkolben eingewogen und mit 25 ml deionisiertem Wasser 24 h bei Zimmertemperatur (25 °C) extrahiert. Das abfiltrierte Granulat wird getrocknet und zurückgewogen.

**Tabelle 4: Darstellung der Ergebnisse des Tests auf Ausbluten**

| Probe | PG Gehalt [Gew-%] | Einwaage [g] | Auswaage [g] | Gewichtsverlust [Gew-%] |
|---|---|---|---|---|
| ABS GP22 | - | 1,0018 | 1,0006 | 0,12 |
| SAN MA VT2421 | - | 1,0104 | 1,0089 | 0,15 |
| A1-1 | 1 | 0,9984 | 0,9985 | <0,01 |
| A2-1 | 1 | 0,9715 | 0,9696 | 0,20 |
| A2-2 | 5 | 1,0130 | 1,0126 | 0,04 |

Der Gewichtsverlust der Proben liegt im Bereich der Eigenfeuchte des Granulats. Ein Ausbluten des Polyguanidins kann daher nicht beobachtet werden.

Probe A1-2, die 1:5 Verdünnung mit Terluran® GP-22 des 5 Gew-% SAN MA Masterbatches, wird einem Langzeittest auf Ausbluten unterworfen. Die Probe wird dazu für 72 h mit Wasser und mit Isopropanol extrahiert.

**Tabelle 5: Darstellung der Gewichtsverluste**

| Probe | PG-Gehalt [Gew-%] | Einwaage [g] | Auswaage [g] | Gewichtsverlust [Gew-%] |
|---|---|---|---|---|
| ABS GP22 | - | 1,1008 | 0,9982 | 0,16 (Isopropanol) |
| SAN MA VT2421 | - | 1,0079 | 1,0057 | 0,22 (Isopropanol) |
| A1-2 | 1 | 1,0300 | 1,0279 | 0,204 (Isopropanol) |
| A1-2 | 1 | 1,0117 | 1,0113 | 0,04 (H₂O) |

Ein Verblenden des SAN MA Masterbatches mit Terluran® GP-22 zeigt ebenfalls kein Ausbluten des Polyguanidins. Der Gewichtsverlust liegt im Rahmen der Eigenfeuchte des Granulats.

### Schmelze-Volumenfließrate-(MVR-)Bestimmung

**Tabelle 6: Darstellung der Schmelze-Volumenfließrate (MVR)**

| Probe | PG-Gehalt | MVR [cm³/10min] |
|---|---|---|
| A1 | 5 | 24,7 ± 0,39 |
| A1-1 | 1 | nicht gemessen |
| A1-2 | 1 | 20,1 ± 0,25 |
| A1-3 | 1 | 18,4 ± 0,10 |
| A2 | 10 | nicht gemessen |
| A2-1 | 1 | nicht gemessen |
| A2-2 | 5 | nicht gemessen |
| A2-3 | 5 | 3,13 ± 0,59 |
| A2-4 | 1 | 15,4 ± 0,18 |
| A3 | 15 | nicht gemessen |
| B1 | 15 | 81,2 ± 5,67 |
| B1-1 | 1 | 19,1 ± 0,39 |
| B1-2 | 5 | 21,0 ± 0,56 |
| B1-3 | 10 | 37,5 ± 2,59 |
| B2 | 25 | 202,0 ± 4,61 |
| Terluran® GP-22 | | 18,3 ± 0,46 |
| SAN MA VT2421 | | 53,1 ± 0,77 |

Die MVR-Werte der Masterbatche sind höher als die des reinen ABS (Terluran® GP-22, Hersteller: INEOS Styrolution, Frankfurt). Die Werte der Compounds nähern sich mit sinkendem PG-Gehalt deutlich den Werten des ABS an.

Aus den antimikrobiell ausgerüsteten ABS-Formmassen werden verschiedene Formteile hergestellt, z.B. Haushaltsgegenstände, die für einen dauerhaften hygienischen Einsatz geeignet sind.

### Zugabe von Polyguanidin während der Extrusion

In einem weiteren Ausführungsbeispiel wurde einer Mischung von ABS (Terluran) mit Polyhexamethylenguanidin (PHMG)-Compatibilizer-Masterbatch zusätzlich während der Extrusion noch PHMG zugesetzt.

Zunächst wurde das Granulat aus ABS und SAN-MA Masterbatch vorgemischt. (Terluran GP22 [ABS] + 20 Gew-% des Polyhexamethylenguanidin (PHMG)-SAN MA-Masterbatches mit 10 Gew-% PHMG). Dann wurde der Extruder hochgefahren, indem in Stufen die Zudosierung an Granulat und parallel dazu die Drehzahl des Extruders erhöht wurde. Nach Erreichen von Druck und Drehmomentkonstanz wurde dann die PHMG-Lösung (3,6 mL/min) zudosiert.
Die auf 50°C temperierte Polyguanidin-Lösung wurde mit Hilfe einer Masterflex-Schlauchpumpe durch eine Düse in die erste Seitenöffnung des Extruders eingeleitet und durch einen Entgasungsdom der entstehende Wasserdampf entfernt. Für 4 Gew-% zusätzliches PHMG wurden bei einem angestrebten Materialdurchsatz von ca. 4 kg/h ca. 167 g/h PG bzw. ca. 207 g/h PG Lösung (-70 Gew-% PG) benötigt. Basierend auf Vorversuchen wurde die Masterflexpumpe auf den Förderwert 3,6 mL/min voreingestellt und der Materialdurchsatz durch Wägeversuche ermittelt. Der erhaltene Strang war flexibel, blassgelb und gut zu granulieren.

Von der hergestellten Zusammensetzung wurden Tests auf antimikrobielle Wirksamkeit, Schmelze Volumenfließrate (MVR) und Ausblutungsmessungen (Leaching-Messungen) durchgeführt. Bei der Extraktion mit Wasser zeigte die Zusammensetzung kein signifikantes Ausbluten (Leaching). Die Ergebnisse liegen im Rahmen der Messungenauigkeit.

Die Tests auf antimikrobielle Wirksamkeit wurden von QualityLabs BT GmbH, Nürnberg, durchgeführt. Die Untersuchungen wurden nach dem Standard Operating Protokoll "QualiScreen: zum Screening antimikrobieller Wirksamkeit von Werkstoffoberflächen" (SOP 3.5 vom 20.11.2015) und in Anlehnung an das Standard Operating Protokoll "Assay zur Bestimmung antimikrobieller Wirksamkeit von Werkstoffoberflächen gegen Staphylococcus epidermidis" (SOP 3.2 vom 20.11.2015) der QualityLabs BT GmbH durchgeführt und überwacht. Ein solches Verfahren ist auch in Fachveröffentlichungen beschrieben (vgl. etwa Bechert et al., 2000, "A new method for screening antiinfective biomaterials", Nat. Med. 6(8):1053-1056; Bechert et al., 1999, "The Erlanger Silver Catheter; In vitro Results for Antimicrobial Activity", Infection 27 Suppl. 1, S24-S29; Kühn, 2010: "Part 2: Effectiveness of a novel gentamicinpalmitate coating on biofilm formation of Staphylococcus areus and Staphylococcus epidermidis", Int. J. Nano and Biomaterials 3(1):107-117; Bruenke et al., 2016, "Quantitative Comparison of the Antimicrobial Efficiency of Leaching versus Nonleaching Polymer Materials", Macromol. Biosci. 16:647-654).

### Prüfbeschreibung des Tests auf antimikrobielle Wirksamkeit:

Die Prüfkörper wurden mit Zellen des Teststamms inkubiert. Loses Zellmaterial, welches nicht auf der Oberfläche adhäriert, wurde durch definierte Waschschritte entfernt. Dem zu testenden Material wurde Zeit gegeben, innerhalb von 18 Stunden (Challenge-Time) bei 37°C Keime auf der Oberfläche auf Grund seiner antimikrobiellen Eigenschaften an der Proliferation (Vermehrung) zu hindern. Die Prüfungsergebnisse beziehen sich somit auf diesen Zeitraum. Wurden alle Bakterien auf der Oberfläche an der Vermehrung gehindert, entwickelten sich keine Tochterzellen und der Prüfkörper verhielt sich "bakterizid". Antimikrobiell konnten sich aber auch solche Werkstoffe verhalten, die weniger als 100% aller Zellen auf der Testoberfläche am Wachstum hinderten. Einige Zellen konnten sich dann vermehren und geben noch Tochterzellen an die Umgebung ab. Dort wurden sie in Gestalt einer Wachstumskurve optisch erfasst. Wenn man die überlebenden Tochterzellen über 48 h kontrolliert vermehrte (Beobachtungszeitraum) konnte eine stärkere Trübung und damit ein größeres Signal erzeugt werden. Gleichzeitig wurden so nur vermehrungsfähige Zellen berücksichtigt.
Besonders antimikrobielle Proben gaben weniger Zellen an die Umgebung ab. Dadurch kam es erst mit deutlicher Verspätung zum mikrobiellen Wachstum. Man beobachtete eine Rechtsverschiebung ("Shift") der Kurve hin zu größeren Zeiten. Die Größe des Shifts ist charakteristisch für das antimikrobielle Verhalten des getesteten Prüfkörpers. Als quantifizierbarer Parameter diente die sogenannte Onset-OD. Das ist die für die überlebenden Tochterzellen erforderliche Stundenzahl, um schließlich zu einer Zellkultur bestimmter optischer Dichte (0.2 OD) heranzuwachsen. Antimikrobielle Wirksamkeit wird immer im Vergleich zu einer nicht-antimikrobiellen so genannten Nullprobe gemessen. Der gemittelte Messwert der Nullprobe wurde vom Messwert der eigentlichen Proben abgezogen (Netto-Onset-OD). Ein Werkstoff wurde nur dann als antimikrobiell im Sinne der QualiScreen-Messung betrachtet, wenn es ihm im Beobachtungszeitraum gelang, im Vergleich zur Nullprobe die Bildung von mindestens 99.9% der Tochterzellen (entspricht einer Netto Onset OD von mindestens 6h) zu verhindern.

**Tabelle 7. Darstellung der Ergebnisse der antimikrobiellen Tests**

| Probe | PG Gehalt [Gew-%] | Teststamm, initiale Keimzahl 5x10⁶ | Onset-OD [h] brutto | Onset-OD [h] netto | CV [Gew-%] | Prüfergebnis |
|---|---|---|---|---|---|---|
| Nullprobe | 0 | Staphylococcus aureus DSM 21979/EDCC 5247 | 6,0 | 24,8 | | Nullprobe |
| A4 | 6 | Staphylococcus aureus DSM 21979/EDCC 5247 | >48,0 | >48,0 | 0,0 | antimikrobiell |
| Nullprobe | 0 | Escherichia coli DSM 1576/ATCC 8739 | 1,4 | - | 2,6 | Nullprobe |
| A4 | 6 | Escherichia coli DSM1576/A TCC 8739 | 26,0 | 24,6 | 97,9 | antimikrobiell |

**Tabelle 8. Darstellung der Ergebnisse des Tests auf Ausbluten**

| Probe | PG Gehalt [Gew-%] | Einwaage [g] | Auswaage [g] | Gewichtsverlust [Gew-%] |
|---|---|---|---|---|
| A4 | 6 | 50,0234 | 50,0144 | 0,0018 |

**Tabelle 9. Darstellung der Schmelze-Volumenfließrate (MVR)**

| Probe | PG-Gehalt | MVR [cm³/10min] |
|---|---|---|
| A4 | 6 | 2,24 ± 0,04 |

Überraschend wurde gefunden, dass insgesamt im Endprodukt ein hoher Anteil von 6 Gew-% PHMG eingebaut werden konnte, wofür nur eine Compatibilizer-Menge (Styrol-Acrylnitril-Maleinsäureanhydrid-Terpolymer) von 18 Gew-% benötigt wurde.

Trotz der geringeren Menge an Compatibilizer zeigt der extrudierte Polymerblend kein Ausbluten. Die antimikrobielle Wirkung ist deutlich vorhanden. Es wird davon ausgegangen, dass übrig gebliebene Anhydrid-Gruppen mit dem zugesetzten PHMG reagieren können, ohne dass es zu einer nennenswerten, unerwünschten Vernetzung der Polymermatrix kommt.

## Patentansprüche

1. Thermoplastische Formmasse M bestehend aus:
(A) 1 bis 100 Gew.-%, insbesondere 2 bis 80 Gew.-%, einer antimikrobiell wirksamen Polymerkomponente A, enthaltend
(A1) 0,2 bis 25 Gew.-%, bezogen auf die Gesamtmasse an Polymer-komponente A, an Polyguanidin als Komponente A1 und
(A2) 75-99,8 Gew.-%, bezogen auf die Gesamtmasse an Polymer-komponente A, an Polymer A2, enthaltend mindestens eine funktionelle Gruppe, welche mit A1 reagieren kann, wobei das Polymer A2 ein Copolymer ist aus:
A21: Maleinsäureanhydrid, Maleinimid und/oder (Meth)acrylsäure, und
A22: einem oder mehreren der Monomere Styrol, Acrylnitril, (Meth)acrylat, und
A23: ggf. einem oder mehreren weiteren mit A21 und A22 copolymerisierbaren Monomeren;
(B) 0 bis 99 Gew.-%, insbesondere 20 bis 98 Gew.-%, einer weiteren thermoplastischen Polymerkomponente B und
(C) 0 bis 40 Gew.-%, insbesondere 0,2 bis 15 Gew.-%, eines oder mehrerer Additive oder Hilfsmittel als Komponente C,
wobei die Summe der Gew.-% der Komponenten A, B und C zusammen 100 Gew.-% ergibt.

2. Thermoplastische Formmasse M gemäß Anspruch 1, wobei das Polymer A2 ein Polymer mit mindestens 1000 Dalton mittlerem Molekulargewicht ist.

3. Thermoplastische Formmasse M gemäß Anspruch 1 und 2, wobei das Polymer A2 eine oder mehrere der folgenden Funktionalitäten enthält: Anhydrid, Säure, Base, Aminogruppe.

4. Thermoplastische Formmasse M gemäß einem der Ansprüche 1 bis 3, wobei Polymer A2 ein Styrol-Acrylnitril-Maleinsäureanhydrid-Terpolymer ist.

5. Thermoplastische Formmasse M gemäß einem der Ansprüche 1 bis 4, wobei die antimikrobiell wirksame Polymerkomponente A 0,2 bis 15 Gew.-%, insbesondere 0,2 bis 10 Gew.-%, bezogen auf die die Gesamtmasse der Polymerkomponente A, an Polyguanidin als Komponente A1 enthält.

6. Thermoplastische Formmasse M gemäß einem der Ansprüche 1 bis 5, wobei das als Komponente A1 eingesetzte Polyguanidin durch Reaktion von Guanidinhydrochlorid (GHC) mit 1,6-Hexamethylendiamin erhalten wird und/oder das als Komponente A1 eingesetzte Polyguanidin ein Molekulargewicht Mₙ von 2000 bis 10.000 aufweist.

7. Thermoplastische Formmasse M gemäß einem der Ansprüche 1 bis 6, wobei das Polymer A2 in einer Menge von 90 bis 99,8 Gew.-%, bezogen auf die Gesamtmasse an Polymerkomponente A, eingesetzt wird, und A2 einen Anteil an Maleinsäure von 0,1 bis 10 Gew.-%, insbesondere 1 bis 5 Gew.-%, bezogen auf die die Gesamtmenge des Polymers A2, aufweist.

8. Thermoplastische Formmasse M gemäß einem der Ansprüche 1 bis 7, wobei die Formmasse M mindestens 20 bis 99 Gew.-% einer thermoplastischen Polymer-komponente B ausgewählt aus der Gruppe bestehend aus Styrol-Copolymeren, Styrol-Butadien-Blockcopolymeren, Polycarbonaten, Polyamiden, Poly(meth)-acrylaten und Polyestern enthält.

9. Thermoplastische Formmasse M gemäß einem der Ansprüche 1 bis 8, wobei die Formmasse M 20 bis 95 Gew.-%, insbesondere 50 bis 95 Gew.-% an Styrol-Copolymer, insbesondere an Acrylnitril-Butadien-Styrol-Copolymer (ABS) als Polymerkomponente B enthält.

10. Thermoplastische Formmasse M gemäß einem der Ansprüche 1 bis 9, wobei die Formmasse M besteht aus:
(A) 1 bis 50 Gew.-%, insbesondere 2 bis 49,8 Gew.-% einer antimikrobiell wirksamen Polymerkomponente A, enthaltend
(A1) 0,2 bis 25 Gew.-%, bezogen auf die Gesamtmasse an Polymer-komponente A, an Polyguanidin als Komponente A1 und
(A2) 75-99,8 Gew.-%, bezogen auf die Gesamtmasse an Polymer-komponente A, an Polymer A2;
(B) 50 bis 99 Gew.-% einer weiteren thermoplastischen Polymerkomponente B, insbesondere Acrylnitril-Butadien-Styrol-Copolymer (ABS) und
(C) 0 bis 10 Gew.-%, insbesondere 0,2 bis 10 Gew.-% eines oder mehrerer Additive oder Hilfsmittel als Komponente C,
wobei die Summe der Gew.-% der Komponenten A, B und C zusammen 100 Gew.-% ergibt.

11. Thermoplastische Formmasse M gemäß einem der Ansprüche 1 bis 10, wobei mindestens 50 Gew.-% der in der Formmasse M enthaltenen polymeren Komponenten amorphe Polymere sind.

12. Verfahren zur Herstellung einer antimikrobiell wirksamen thermoplastischen Formmasse M enthaltend die folgenden Verfahrensschritte:
(i) Bereitstellen der folgenden Komponenten:
(A1) Polyguanidin als Komponente A1 und
(A2) Polymer A2, enthaltend mindestens eine funktionelle Gruppe, welche mit A1 reagieren kann; und optional
(B) weiterer thermoplastischer Komponente(n) B und optional
(C) eines oder mehrerer Additive oder Hilfsmittel als Komponente C;
(ii) Extrudieren einer Mischung der Komponenten A1 und A2 und optional C zu einer antimikrobiell wirksamen, thermoplastischen Polymerkomponente A und
(iii) Extrudieren der aus Schritt (ii) erhaltenen antimikrobiell wirksamen Polymerkomponente A mit einer oder mehreren weiteren thermoplastischen Polymerkomponenten B und/oder einem oder mehreren Additiven oder Hilfsmitteln als Komponente C, wobei während dem Schritt (iii) Polyguanidin als Komponente A1 zugesetzt wird.

13. Verfahren gemäß Anspruch 12, wobei die thermoplastische Formmasse eine polymere Formmasse M gemäß einem der Ansprüche 1 bis 11 ist.

14. Formteil F, enthaltend mindestens eine thermoplastische Formmasse M gemäß einem der Ansprüche 1 bis 11 oder mindestens eine thermoplastische Formmasse M erhältlich nach einem Verfahren gemäß einem der Ansprüche 12 oder 13, insbesondere hergestellt durch Extrudieren, Spritzgießen, Blasformen, Verspinnen oder einen Sprühvorgang.

15. Produkt bestehend aus oder enthaltend mindestens ein(em) Formteil F gemäß Anspruch 14, insbesondere wobei das Produkt ausgewählt ist aus der Gruppe von: Fahr- und Flugzeugteilen, Schiffsteilen, Verpackungen, Rohren, Schläuchen, Sanitärartikeln, Medizinprodukten, Eingabegeräten und Bedienelementen, Laborgeräten- und Verbrauchsgütern, Maschinenteilen, Haushaltsgeräten, Mobiliar, Griffen, Dichtungen, Bodenbelägen, Textilien, Agrargeräten, Schuhsohlen, Gefäßen zur Aufbewahrung von Nahrungs- und Futtermitteln, Geschirr, Besteck, Filtern, Fernsprechgeräten und Beschichtungen.

16. Verwendung einer thermoplastischen Formmasse M gemäß einem der Ansprüche 1 bis 11 oder einer Formmasse M erhältlich nach einem Verfahren gemäß einem der Ansprüche 12 oder 13 zur Herstellung eines antimikrobiellen Formteils F zur Verhinderung von mikrobiellem Wachstum, insbesondere zur Verhinderung der Ausbildung eines Bio-Films, auf der Oberfläche von oder in dem Formteil F.

17. Verfahren zur Verhinderung von mikrobiellem Wachstum, insbesondere zur Verhinderung der Ausbildung eines Bio-Films, auf der Oberfläche von oder in dem Formteil F, umfassend die folgenden Schritte:
(i) Bereitstellen einer thermoplastischen Formmasse M gemäß einem der Ansprüche 1 bis 11 oder einer Formmasse M erhältlich nach einem Verfahren gemäß einem der Ansprüche 12 oder 13 und
(ii) Herstellen des Formteils F, insbesondere durch Extrudieren, Spritzgießen, Blasformen, Verspinnen oder einen Sprühvorgang.

## Claims

1. Thermoplastic moulding composition M consisting of:
(A) 1 to 100 wt%, especially 2 to 80 wt%, of an antimicrobially active polymer component A, comprising
(A1) 0.2 to 25 wt%, based on the total mass of polymer component A, of polyguanidine as component A1, and
(A2) 75-99.8 wt%, based on the total mass of polymer component A, of polymer A2, comprising at least one functional group which is able to react with A1, the polymer A2 being a copolymer of:
A21: maleic anhydride, maleimide and/or (meth)acrylic acid, and
A22: one or more of the monomers styrene, acrylonitrile, (meth)acrylate, and
A23: optionally one or more further monomers copolymerizable with A21 and A22;
(B) 0 to 99 wt%, especially 20 to 98 wt%, of a further thermoplastic polymer component B; and
(C) 0 to 40 wt%, especially 0.2 to 15 wt%, of one or more additives or auxiliaries as component C;
where the sum total of the wt% of components A, B and C together is 100 wt%.

2. Thermoplastic moulding composition M according to Claim 1, the polymer A2 being a polymer of at least 1000 daltons average molecular weight.

3. Thermoplastic moulding composition M according to Claim 1 and 2, the polymer A2 comprising one or more of the following functionalities: anhydride, acid, base, amino group.

4. Thermoplastic moulding composition M according to any of Claims 1 to 3, polymer A2 being a styreneacrylonitrile-maleic anhydride terpolymer.

5. Thermoplastic moulding composition M according to any of Claims 1 to 4, the antimicrobially active polymer component A comprising 0.2 to 15 wt%, especially 0.2 to 10 wt%, based on the total mass of the polymer component A, of polyguanidine as component A1.

6. Thermoplastic moulding composition M according to any of Claims 1 to 5, the polyguanidine used as component A1 being obtained by reaction of guanidine hydrochloride (GHC) with 1,6-hexamethylenediamine and/or the polyguanidine used as component A1 having a molecular weight Mₙ of 2000 to 10 000.

7. Thermoplastic moulding composition M according to any of Claims 1 to 6, the polymer A2 being used in an amount of 90 to 99.8 wt%, based on the total mass of polymer component A, and A2 having a maleic acid fraction of 0.1 to 10 wt%, especially 1 to 5 wt%, based on the total amount of the polymer A2.

8. Thermoplastic moulding composition M according to any of Claims 1 to 7, the moulding composition M comprising at least 20 to 99 wt% of a thermoplastic polymer component B selected from the group consisting of styrene copolymers, styrene-butadiene block copolymers, polycarbonates, polyamides, poly(meth)acrylates and polyesters.

9. Thermoplastic moulding composition M according to any of Claims 1 to 8, the moulding composition M comprising 20 to 95 wt%, especially 50 to 95 wt%, of styrene copolymer, especially of acrylonitrile-butadiene-styrene copolymer (ABS) as polymer component B.

10. Thermoplastic moulding composition M according to any of Claims 1 to 9, the moulding composition M consisting of:
(A) 1 to 50 wt%, especially 2 to 49.8 wt%, of an antimicrobially active polymer component A, comprising
(A1) 0.2 to 25 wt%, based on the total mass of polymer component A, of polyguanidine as component A1, and
(A2) 75-99.8 wt%, based on the total mass of polymer component A, of polymer A2;
(B) 50 to 99 wt% of a further thermoplastic polymer component B, especially acrylonitrile-butadiene-styrene copolymer (ABS); and
(C) 0 to 10 wt%, especially 0.2 to 10 wt%, of one or more additives or auxiliaries as component C;
the sum total of the wt% of components A, Band C together being 100 wt%.

11. Thermoplastic moulding composition M according to any of Claims 1 to 10, at least 50 wt% of the polymeric components comprised in the moulding composition M being amorphous polymers.

12. Process for producing an antimicrobially active thermoplastic moulding composition M, comprising the following process steps:
(i) providing the following components:
(A1) polyguanidine as component A1 and
(A2) polymer A2 comprising at least one functional group which is able to react with A1; and optionally
(B) further thermoplastic component(s) B and optionally
(C) one or more additives or auxiliaries as component C;
(ii) extruding a mixture of components A1 and A2 and optionally C to give an antimicrobially active thermoplastic polymer component A and
(iii) extruding the antimicrobially active polymer component A, obtained from step (ii), with one or more further thermoplastic polymer components B and/or one or more additives or auxiliaries as component C, polyguanidine being added as component A1 during step (iii) .

13. Process according to Claim 12, the thermoplastic moulding composition being a polymeric moulding composition M according to any of Claims 1 to 11.

14. Moulding F comprising at least one thermoplastic moulding composition M according to any of Claims 1 to 11 or at least one thermoplastic moulding composition M obtainable by a process according to either of Claims 12 and 13, especially produced by extruding, injection moulding, blow moulding, spinning or a spraying procedure.

15. Product consisting of or comprising at least one moulding F according to Claim 14, especially where the product is selected from the group of: vehicle and aircraft parts, ship parts, packaging, pipes, hoses, sanitary items, medical products, input devices and control elements, laboratory equipment and consumer goods, machine parts, household appliances, furniture, handles, gaskets, floorcoverings, textiles, agricultural equipment, footwear soles, vessels for the storage of foodstuffs and animal feed, crockery, cutlery, filters, telephone equipment and coatings.

16. Use of a thermoplastic moulding composition M according to any of Claims 1 to 11 or of a moulding composition M obtainable by a process according to either of Claims 12 and 13 for producing an antimicrobial moulding F for preventing microbial growth, especially for preventing the development of a biofilm, on the surface of or in the moulding F.

17. Process for preventing microbial growth, especially for preventing the development of a biofilm, on the surface of or in the moulding F, comprising the following steps:
(i) providing a thermoplastic moulding composition M according to any of Claims 1 to 11 or a moulding composition M obtainable by a process according to either of Claims 12 and 13, and
(ii) producing the moulding F, especially by extruding, injection moulding, blow moulding, spinning or a spraying procedure.

## Revendications

1. Matériau de moulage thermoplastique M constitué par :
(A) 1 à 100 % en poids, notamment 2 à 80 % en poids, d'un composant polymère à activité antimicrobienne A, contenant :
(A1) 0,2 à 25 % en poids, par rapport à la masse totale du composant polymère A, de polyguanidine en tant que composant A1, et
(A2) 75 à 99,8 % en poids, par rapport à la masse totale du composant polymère A, d'un polymère A2, contenant au moins un groupe fonctionnel qui peut réagir avec A1, le polymère A2 étant un copolymère constitué par :
A21 : de l'anhydride de l'acide maléique, du maléinimide et/ou de l'acide (méth)acrylique, et
A22 : un ou plusieurs des monomères styrène, acrylonitrile, (méth)acrylate, et
A23 : éventuellement un ou plusieurs monomères supplémentaires, copolymérisables avec A21 et A22 ;
(B) 0 à 99 % en poids, notamment 20 à 98 % en poids, d'un composant polymère thermoplastique supplémentaire B, et
(C) 0 à 40 % en poids, notamment 0,2 à 15 % en poids, d'un ou de plusieurs additifs ou adjuvants en tant que composant C,
la somme des % en poids des composants A, B et C ensemble étant de 100 % en poids.

2. Matériau de moulage thermoplastique M selon la revendication 1, dans lequel le polymère A2 est un polymère ayant un poids moléculaire moyen d'au moins 1 000 Dalton.

3. Matériau de moulage thermoplastique M selon les revendications 1 et 2, dans lequel le polymère A2 contient une ou plusieurs des fonctionnalités suivantes : anhydride, acide, base, groupe amino.

4. Matériau de moulage thermoplastique M selon l'une quelconque des revendications 1 à 3, dans lequel le polymère A2 est un terpolymère de styrène-acrylonitrile-anhydride de l'acide maléique.

5. Matériau de moulage thermoplastique M selon l'une quelconque des revendications 1 à 4, dans lequel le composant polymère à activité antimicrobienne A contient 0,2 à 15 % en poids, notamment 0,2 à 10 % en poids, par rapport à la masse totale du composant polymère A, de polyguanidine en tant que composant A1.

6. Matériau de moulage thermoplastique M selon l'une quelconque des revendications 1 à 5, dans lequel la polyguanidine utilisée en tant que composant A1 est obtenue par réaction de chlorhydrate de guanidine (GHC) avec de la 1,6-hexaméthylène-diamine, et/ou la polyguanidine utilisée en tant que composant A1 présente un poids moléculaire Mₙ de 2 000 à 10 000.

7. Matériau de moulage thermoplastique M selon l'une quelconque des revendications 1 à 6, dans lequel le polymère A2 est utilisé en une quantité de 90 à 99, 8 % en poids, par rapport à la masse totale du composant polymère A, et A2 présente une proportion d'acide maléique de 0,1 à 10 % en poids, notamment de 1 à 5 % en poids, par rapport à la quantité totale du polymère A2.

8. Matériau de moulage thermoplastique M selon l'une quelconque des revendications 1 à 7, dans lequel le matériau de moulage M contient au moins 20 à 99 % en poids d'un composant polymère thermoplastique B choisi dans le groupe constitué par les copolymères de styrène, les copolymères séquencés de styrène-butadiène, les polycarbonates, les polyamides, les poly(méth)acrylates et les polyesters.

9. Matériau de moulage thermoplastique M selon l'une quelconque des revendications 1 à 8, dans lequel le matériau de moulage M contient 20 à 95 % en poids, notamment 50 à 95 % en poids, d'un copolymère de styrène, notamment d'un copolymère d'acrylonitrile-butadiène-styrène (ABS) en tant que composant polymère B.

10. Matériau de moulage thermoplastique M selon l'une quelconque des revendications 1 à 9, dans lequel le matériau de moulage M est constitué par :
(A) 1 à 50 % en poids, notamment 2 à 49,8 % en poids, d'un composant polymère à activité antimicrobienne A, contenant :
(A1) 0,2 à 25 % en poids, par rapport à la masse totale du composant polymère A, de polyguanidine en tant que composant A1, et
(A2) 75 à 99,8 % en poids, par rapport à la masse totale du composant polymère A, d'un polymère A2 ;
(B) 50 à 99 % en poids d'un composant polymère thermoplastique supplémentaire B, notamment un copolymère d'acrylonitrile-butadiène-styrène (ABS), et
(C) 0 à 10 % en poids, notamment 0,2 à 10 % en poids, d'un ou de plusieurs additifs ou adjuvants en tant que composant C,
la somme des % en poids des composants A, B et C ensemble étant de 100 % en poids.

11. Matériau de moulage thermoplastique M selon l'une quelconque des revendications 1 à 10, dans lequel au moins 50 % en poids des composants polymères contenus dans le matériau de moulage M sont des polymères amorphes.

12. Procédé de fabrication d'un matériau de moulage thermoplastique à activité antimicrobienne M, contenant les étapes de procédé suivantes :
(i) la mise à disposition des composants suivants :
(A1) de la polyguanidine en tant que composant A1, et
(A2) un polymère A2, contenant au moins un groupe fonctionnel qui peut réagir avec A1 ; et éventuellement
(B) un ou plusieurs composants thermoplastiques supplémentaires B, et éventuellement
(C) un ou plusieurs additifs ou adjuvants en tant que composant C ;
(ii) l'extrusion d'un mélange des composants A1 et A2 et éventuellement C en un composant polymère thermoplastique à activité antimicrobienne A, et
(iii) l'extrusion du composant polymère à activité antimicrobienne A obtenu à l'étape (ii) avec un ou plusieurs composants polymères thermoplastiques supplémentaires B et/ou un ou plusieurs additifs ou adjuvants en tant que composant C, de la polyguanidine étant ajoutée en tant que composant A1 pendant l'étape (iii).

13. Procédé selon la revendication 12, dans lequel le matériau de moulage thermoplastique est un matériau de moulage polymère M selon l'une quelconque des revendications 1 à 11.

14. Pièce moulée F, contenant au moins un matériau de moulage thermoplastique M selon l'une quelconque des revendications 1 à 11 ou au moins un matériau de moulage thermoplastique M pouvant être obtenu par un procédé selon l'une quelconque des revendications 12 ou 13, notamment fabriquée par extrusion, moulage par injection, moulage par soufflage, filage ou un processus de pulvérisation.

15. Produit constitué par ou contenant au moins une pièce moulée F selon la revendication 14, le produit étant notamment choisi dans le groupe constitué par : les pièces de véhicules et d'aéronefs, les pièces de navires, les emballages, les tubes, les tuyaux, les articles sanitaires, les produits médicaux, les appareils de saisie et les éléments de commande, les appareils et produits de consommation de laboratoire, les pièces de machines, les appareils ménagers, les meubles, les poignées, les joints, les revêtements de sol, les textiles, les appareils agricoles, les semelles de chaussures, les contenants pour la conservation de produits alimentaires et d'aliments pour animaux, la vaisselle, les couverts, les filtres, les appareils téléphoniques et les revêtements.

16. Utilisation d'un matériau de moulage thermoplastique M selon l'une quelconque des revendications 1 à 11 ou d'un matériau de moulage M pouvant être obtenu par un procédé selon l'une quelconque des revendications 12 ou 13, pour la fabrication d'une pièce moulée antimicrobienne F pour la prévention de la croissance microbienne, notamment pour la prévention de la formation d'un biofilm, sur la surface de ou dans la pièce moulée F.

17. Procédé de prévention de la croissance microbienne, notamment de prévention de la formation d'un biofilm, sur la surface de ou dans la pièce moulée F, comprenant les étapes suivantes :
(i) la mise à disposition d'un matériau de moulage thermoplastique M selon l'une quelconque des revendications 1 à 11 ou d'un matériau de moulage M pouvant être obtenu par un procédé selon l'une quelconque des revendications 12 ou 13, et
(ii) la fabrication de la pièce moulée F, notamment par extrusion, moulage par injection, moulage par soufflage, filage ou un processus de pulvérisation.
